# EUROPEAN PATENT APPLICATION

(11) **EP 4 591 873 A1**
(43) Date of publication of application: **30.07.2025**
(21) Application number: 23868598.6
(22) Date of filing: 20.09.2023
(51) Int. Cl.: A61K 35/747, A61K 45/06, A61P 35/00, A23L 33/135

(54) **COMBINED THERAPY OF LACTOBACILLUS PLANTARUM STRAIN, AND CANCER TREATMENT METHOD USING SAME**

(30) Priority: 20.09.2022 KR 20220118924; 10.02.2023 KR 20230017958; 14.07.2023 KR 20230091864
(71) Applicant: Gi Biome Inc., Seongnam-si, Gyeonggi-do 13201 (KR); GI Innovation, Inc., Seoul 05855 (KR)
(72) Inventor: JANG, Myung Ho, Seoul 05849 (KR); YANG, Bo Gie, Seoul 05849 (KR); KIM, A Ram, Namyangju-si, Gyeonggi-do 12095 (KR); KOH, Young Jun, Seoul 05855 (KR)
(74) Representative: Vossius & Partner Patentanwälte Rechtsanwälte mbB
(86) International application number: PCT/KR2023/014314
(87) International publication number: WO 2024/063546

(57) **Abstract**

The present disclosure relates to a pharmaceutical composition for treating cancer, including a *Lactobacillus plantarum* strain and an anticancer agent which are co-administered. The *Lactobacillus plantarum* strain, or a culture broth of the strain not only can inhibit the growth of cancer cells, but can also induce the apoptosis of cancer cells, and when administered in combination with an anticancer agent, exhibits a synergistic effect on inhibition of the growth of cancer cells and tumors, and thus can be effectively used to prevent, treat or ameliorate cancer in the form of a pharmaceutical composition or health functional food.

## Description

### Technical Field

The present disclosure relates to a composition for preventing or treating cancer by combined therapy, the composition including a *Lactobacillus plantarum* strain and an anticancer agent.

### Background Art

Microbiome is a compound word of microbe and genome, and human microbiome refers to the genome of all microorganisms living in the human body. Several parts of the human body consist of various types of microorganisms. 70 % of the human microbiome is distributed in the digestive tract, with the largest number of microorganisms living in the large intestine. Since the microbiome is closely related to the human immune system and physical development, a high correlation with various diseases, including metabolic diseases such as obesity and diabetes, inflammatory bowel disease, depression, and the like, has been consistently reported. After the Human genome project was initially completed in 2002, attempts were made to confirm the correlation between genes and incurable diseases, but it was confirmed that environmental factors, especially the microbiome environment, may have a greater impact on diseases. Microbiome therapeutic agents are living organisms that proliferate in the intestines and affect the human body through interactions with human cells. For this reason, the value thereof as innovative new drugs capable of improving the low efficacy and high recurrence rate of existing drugs for incurable diseases is attracting great attention. In addition, as the U.S. FDA approved in December 2022 REBYOTA^{®} from Ferring Pharmaceuticals, which is the first microbiome-based therapeutic agent for Clostridium difficile (C. difficile) infection (CDI), microbiome therapeutic agents started to be commercially available. Also, "SER-109" from Seres Therapeutics is under FDA review as the first oral microbiome therapeutic agent.

Worldwide, cancer is considered one of the major health problems. Cancer is the second leading cause of death in the world. Once cancer develops, it requires intensive treatment and consistent management. Therefore, cancer is a disease with a very high burden on individuals, society and economy. As of 2021, the global anticancer agent market is approximately 225 trillion won. Due to the increase in the number of patients, the development of cancer immunotherapeutic agents, the emergence of new therapies, and the like, the market is expected to grow at an average annual rate of 11 % in the future, which will reach approximately 470 trillion won in 2028.

Conventional cancer treatments involve a combination of chemotherapy, surgery, hormonal therapy, and/or radiotherapy to eradicate neoplastic cells in a patient. Cancer chemotherapy is based on the use of drugs which kill replicating cells, preferably faster than agents kill the patient's normal cells. Anticancer agents intervene in the metabolic pathway of cancer cells to block the process of replication, transcription, and translation of DNA through direct interaction with DNA, or interfere with the synthesis of nucleic acid precursors, and inhibit cell division, thereby showing cytotoxicity to cells. Accordingly, these anticancer agents do not selectively act only on cancer cells, but cause fatal damage to normal cells, particularly tissue cells in which cell division is actively performed, resulting in a variety of severe side effects such as vomiting, bone marrow function degradation, gastrointestinal disorders, alopecia, diarrhea, and liver and kidney toxicity. As an alternative, cancer immunotherapeutic agents (or immune checkpoint inhibitors) are receiving great attention as third-generation anticancer agents, but the requirements for their use are very limited. Treatment effects are also generally only seen in less than 30 % of cases. Thus, to completely conquer the disease called cancer, it is necessary to pay more attention to studies on the mechanisms by which intestinal microorganisms enhance the efficacy of cancer immunotherapeutic agents.

Recently, combined therapies that enhance the effectiveness of treatment for diseases have emerged. Therefore, there is a need to develop combined therapy capable of synergistically increasing the effect of treatment using a single ingredient.

### Disclosure of Invention

### Technical Problem

An aspect provides a pharmaceutical composition for preventing or treating cancer, which: includes, as active ingredients, a first active substance including a *Lactobacillus plantarum* strain, a culture of the strain, a lysate of the strain, or a mixture thereof, and a second active substance including a cancer immunotherapeutic agent as a first anticancer agent; or includes the first active substance as an active ingredient, and is administered in combination with the second active substance.

Another aspect provides a health functional food for preventing or ameliorating cancer, which: includes, as active ingredients, a first active substance including a *Lactobacillus plantarum* strain, a culture of the strain, a lysate of the strain, or a mixture thereof, and a second active substance including a cancer immunotherapeutic agent as a first anticancer agent; or includes the first active substance as an active ingredient, and is administered in combination with the second active substance.

Another aspect provides a kit for preventing or treating cancer, which: includes, as active ingredients, a first active substance including a *Lactobacillus plantarum* strain, a culture of the strain, a lysate of the strain, or a mixture thereof, and a second active substance including a cancer immunotherapeutic agent as a first anticancer agent; or includes the first active substance as an active ingredient, and is administered in combination with the second active substance.

Another aspect provides a method of delivering a drug into a subject, including administering a composition which: includes, as active ingredients, a first active substance including a *Lactobacillus plantarum* strain, a culture of the strain, a lysate of the strain, or a mixture thereof, and a second active substance including a cancer immunotherapeutic agent as a first anticancer agent; or includes the first active substance as an active ingredient, and is administered in combination with the second active substance, to a subject in need thereof.

Another aspect provides a method of preventing or treating cancer, including administering an effective amount of a composition which: includes, as active ingredients, a first active substance including a *Lactobacillus plantarum* strain, a culture of the strain, a lysate of the strain, or a mixture thereof, and a second active substance including a cancer immunotherapeutic agent as a first anticancer agent; or includes the first active substance as an active ingredient, and is administered in combination with the second active substance, to a subject in need thereof.

Another aspect provides use of: a composition including, as active ingredients, a first active substance including a *Lactobacillus plantarum* strain, a culture of the strain, a lysate of the strain, or a mixture thereof, and a second active substance including a cancer immunotherapeutic agent as a first anticancer agent; or a composition including the first active substance as an active ingredient, and administered in combination with the second active substance, for preparing a pharmaceutical formulation or health functional food for preventing or treating cancer.

### Technical Solution

An aspect provides a strain of the genus *Lactobacillus,* particularly a *Lactobacillus plantarum* GB104 strain, with anticancer activity, for example, anticancer activity against colorectal cancer.

*Lactobacillus* is an aerobic or facultative anaerobic, gram-positive *bacillus* widely distributed in nature. Microorganisms belonging to the genus *Lactobacillus* include *Lactobacillus plantarum, Lactobacillus sakei,* and the like. As a result of having researched to develop a novel strain with an excellent anticancer effect, the inventors of the present disclosure selected *Lactobacillus plantarum* GB104 as an anticancer candidate strain. The strain was deposited in the Korea Collection for Type Cultures of Korea Research Institute of Bioscience and Biotechnology under accession number KCTC14107BP on January 14, 2020. The strain belongs to a probiotic strain, is harmless to the human body, and may be used without side effects.

The name of *Lactobacillus* has been changed to *Limosilactobacillus* or *Lactiplantibacillus,* and the changed strain names in this specification can be used interchangeably. For example, the strain name *Lactobacillus plantarum* was changed to *Lactiplantibacillus plantarum.*

The term *"Lactobacillus plantarum* GB104" as used herein may be interchangeably described as a *L. Plantarum* GB104 strain or a *Lactobacillus plantarum* GB104 strain (Accession No.: KCTC 14107BP).

In an embodiment, the strain may be a strain deposited under accession number KCTC14107BP.

In an embodiment, the strain may be a strain including a 16S rRNA gene consisting of the nucleotide sequence of SEQ ID NO: 1.

In an embodiment, the strain may be a strain having 16S rRNA consisting of the nucleotide sequence of SEQ ID NO: 1 or 16s rRNA including a nucleotide sequence with 97% or more nucleotide sequence identity therewith. Specifically, the strain may have at least 93 %, 95 %, 96 %, 97 %, 98 %, 99 %, 99.5 %, 99.8 %, 99.9 % or 100 % homology to the nucleotide sequence consisting of SEQ ID NO: 1 of this specification.

In an embodiment, the strain may be a mutant of a naturally occurring strain.

In an embodiment, the strain may be a live bacterium, a dead bacterium, or a cytoplasmic fraction obtained by lysing the strain, and may preferably be a live bacterium.

The term "culture" as used herein may be used interchangeably with "culture supernatant," "conditioned culture broth," or "conditioned medium," and may refer to the entire medium including: a stain obtained by culturing a strain of the genus *Lactobacillus* in a medium capable of supplying nutrients to the strain to grow and survive *in vitro* for a certain period of time; metabolites thereof; extra nutrients; and the like. The culture refers to a product obtained by culturing a probiotic strain in a known medium, and the product may or may not include the strain itself. The medium may be selected from known liquid media or solid media, and for example, may be, but is not limited to, an MRS liquid medium, a GAM liquid medium, an MRS agar medium, a GAM agar medium, and a BL agar medium.

The term "lysate" as used herein may be used interchangeably with "homogenate," and may refer to a solution or suspension in an aqueous medium of crushed cells of a microorganism such as *Lactobacillus plantarum.* A cell lysate includes, for example, macromolecules such as DNA, RNA, proteins, peptides, carbohydrates, or lipids and/or micromolecules such as amino acids, sugars, or fatty acids, or fractions thereof. The lysate also includes cell debris, which may be smooth or have a granular structure.

The culture broth may include a culture broth itself obtained by culturing a strain, or a concentrate or freeze-dried product thereof, or a culture supernatant obtained by removing the strain from the culture broth, or a concentrate or freeze-dried product thereof.

The culture broth may be obtained by culturing *Lactobacillus plantarum* in an appropriate medium (e.g., MRS plate medium) at any temperature exceeding 10 °C or less than 40 °C for a certain period of time, for example, 4 hours to 50 hours.

In an embodiment, the first active substance including one or more selected from the group consisting of the strain, a culture of the strain, a lysate of the strain, or an extract of the strain, culture and lysate may have anticancer activity.

Specifically, the anticancer activity may be an activity of delaying the onset of a tumor or inhibiting the growth rate of a tumor.

In an embodiment, the first active substance may have tumor growth inhibitory activity or tumor metastasis inhibitory activity.

In an embodiment, the first active substance may induce apoptosis of cancer cells.

According to an embodiment, the anticancer activity may be a cancer cell growth inhibitory activity in which, when colorectal cancer cells HCT116 were treated with a supernatant of the *Lactobacillus plantarum* GB104 strain, cancer cell viability was reduced by 90 % to 98 %, or may be an activity in which, when the *Lactobacillus plantarum* GB104 strain was administered to mice subcutaneously transplanted with the cancer cell line MC-38 or HT-29, a tumor volume was 10 % to 90 % compared with a control not administered with the strain.

In an embodiment, the anticancer activity may be an activity that induces the apoptosis of cancer cells, and may be an activity in which, when colorectal cancer cells HCT116 were treated with a supernatant of the *Lactobacillus plantarum* GB104 strain, induced initial apoptosis of the cancer cells was increased 5 times to 9 times, compared with a control not administered with the strain.

In an embodiment, the first active substance may have anticancer activity, and specifically, may have anticancer activity against colorectal cancer.

The term "cancer" as used herein typically refers to a physiological condition in an animal, characterized by abnormal or uncontrolled cell growth. For example, cancer and cancer pathology may be related to metastasis, interference with normally functioning surrounding cells, release of cytokines or other secreted products at abnormal levels, inhibition or enhancement of inflammatory or immunological responses, neoplasia, premalignancy, malignancy, invasion into surrounding or distant tissues or organs, such as lymph node, and the like.

The cancer may be gastrointestinal cancer or non-gastrointestinal cancer.

The gastrointestinal cancer is a malignant tumor occurring in the gastrointestinal tract such as esophagus, stomach, small intestine, or large intestine. For example, the gastrointestinal cancer may be, but is not limited to, one or more cancers selected from the group consisting of esophageal cancer, gallbladder cancer, liver cancer, biliary tract cancer, pancreatic cancer, gastric cancer, small intestine cancer, colorectal cancer, colon cancer, anal cancer, and rectal cancer. In an embodiment, the gastrointestinal cancer may be colorectal cancer.

The non-gastrointestinal cancer includes malignant tumors occurring in tissues other than the gastrointestinal tract or digestive system without limitation, and for example, may be, but is not limited to, hematologic malignancy, leukemia, acute myeloid leukemia, neuroblastoma, retinoblastoma, lung cancer, head and neck cancer, salivary gland cancer, melanoma, laryngeal cancer, prostate cancer, breast cancer, bladder cancer, kidney cancer, multiple myeloma, cervical cancer, thyroid cancer, ovarian cancer, urethral cancer, skin cancer, osteosarcoma, glioblastoma, brain tumor, or lymphoma.

In an embodiment, the cancer may be any one selected from the group consisting of gastric cancer, liver cancer, lung cancer, colorectal cancer, breast cancer, prostate cancer, ovarian cancer, pancreatic cancer, gallbladder cancer, biliary tract cancer, cervical cancer, thyroid cancer, laryngeal cancer, acute myeloid leukemia, brain tumor, neuroblastoma, retinoblastoma, salivary gland cancer, melanoma, bladder cancer, kidney cancer, hematologic malignancy, esophageal cancer, head and neck cancer, skin cancer, small intestine cancer, anal cancer, colon cancer, rectal cancer, and lymphoma.

In an embodiment, the colorectal cancer includes cancer occurring in one or more sites selected from the group consisting of ascending colon, transverse colon, descending colon, sigmoid colon, and rectal mucosa. The colorectal cancer may be, but is not limited to, one or more types selected from the group consisting of adenocarcinoma, lymphoma, malignant carcinoid, leiomyosarcoma, Kaposi's sarcoma, and squamous cell carcinoma.

In an embodiment, the first active substance may include the *Lactobacillus plantarum* strain alone as an active ingredient, or may include one or more pharmaceutically acceptable carriers, excipients or diluents.

In an embodiment, the second active substance may include an anticancer agent. More specifically, the second active ingredient may include a first anticancer agent and a second anticancer agent, which are two different types of anticancer agents.

The anticancer agent may be selected from the group consisting of a chemotherapeutic agent for chemotherapy, which is conventional therapy that can be used in combination, a targeted anticancer agent, an anticancer virus, an antibody therapeutic agent, a cancer immunotherapeutic agent (hereinafter, may be used interchangeably with "immune checkpoint inhibitor), and a combination thereof. Specifically, the anticancer agent may include an antibody, an antigen-binding fragment, or a fusion protein including one or more single domain antibodies or antigen-binding fragment(s) thereof and one or more additional polypeptides. For example, the fusion protein may include one or more single domain antibodies and a constant region or Fc region, and the one or more single domain antibodies or antigen-binding fragment(s) thereof may be non-covalently or covalently conjugated to an antigen.

In an embodiment, the second active substance may include a cancer immunotherapeutic agent.

The term "antibody" as used herein refers to any isotype of an intact immunoglobulin or an antigen-binding fragment capable of competing with an intact antibody for binding to a target antigen. For example, the antibody includes chimeric, humanized, fully human and bispecific antibodies or antigen-binding fragments thereof. The antibody itself is one type of an antigen-binding protein. The antibody or antigen-binding fragment thereof may be derived from only one source or a chimera. The chimeric antibody includes portions derived from two different types of antibodies, and will be described below in more detail. The antibody or antigen-binding fragment thereof may be produced by hybridoma, recombinant DNA technology, or enzymatic or chemical cleavage of an intact antibody. Unless otherwise stated, the term antibody as used herein may include antibodies including two full-length heavy chains and two full-length light chains, and derivatives, variants, fragments and mutants thereof.

In the present specification, "light chain" includes a full-length light chain having a sufficient variable region sequence to provide binding specificity for an antigen or epitope and a fragment thereof. The full-length light chain includes a variable region domain VL and a constant region domain CL. The variable region domain of the light chain is present at the amino-terminus of a light-chain polypeptide. Light chains include kappa chains and lambda chains.

In the present specification, "heavy chain" includes a full-length heavy chain having a sufficient variable region sequence to provide binding specificity for an antigen or epitope and a fragment thereof. The full-length heavy chain includes a variable region domain VH and three constant region domains CH1, CH2 and CH3. The VH domain is located at the amino-terminus of a heavy chain polypeptide, and the CH domain is located at the carboxyl-terminus thereof, with CH3 being closest to the carboxy-terminus. The heavy chain includes IgG (including IgG1, IgG2, IgG3, and IgG4 subtypes), IgA (including IgA1 and IgA2 subtypes), and isotypes of IgM and IgE.

The term "antigen-binding fragment" of an antibody or immunoglobulin chain (heavy chain or light chain), as used herein, includes a portion of an antibody that lacks some amino acids compared with a full-length chain but is capable of specifically binding to an antigen. Such fragments may be considered to have biological activity in that they can specifically bind to a target antigen or compete with other antibodies or antigen-binding fragments for binding to a specific epitope. In an aspect, such a fragment includes at least one CDR present in the full-length light or heavy chain, and in some embodiments, such a fragment includes a short heavy chain and/or light chain or a portion thereof. These biologically active fragments may be produced by recombinant DNA techniques and for example, may be produced by enzymatic or chemical cleavage of intact antibodies. Immunologically functional immunoglobulin fragments include, but are not limited to, Fab, Fab', F(ab')2, scFab, dsFv, Fv, scFV, scFV-Fc, a diabody, a minibody, scAb, and dAb, and may be derived from any mammal, including human, mouse, rat, camelid, or rabbit, but the present disclosure is not limited thereto.

In the present disclosure, "Fc" region includes two heavy chain fragments including CH2 and CH3 domains of an antibody. These two heavy chain fragments are linked to each other by hydrophobic interaction of two or more disulfide bonds and a CH3 domain.

In the present disclosure, "Fab fragment" consists of one light chain and one heavy chain, including a variable region and CH1 only. The heavy chain of a Fab molecule cannot form a disulfide bond with another heavy chain molecule. In an scFab, two molecules of Fab are linked by a flexible linker.

In the present disclosure, "Fab' fragment" includes a region between CH1 and CH2 domains of a heavy chain, in addition to Fab fragment. A disulfide bond may be formed between two heavy chains of two molecules of Fab' fragment, to form a F(ab')2 molecule.

In the present disclosure, "F(ab')2 fragment" includes two light chains and, as mentioned above, two heavy chains including a variable region CH1 and part of a constant region between CH1 and CH2 domains, with a disulfide bond formed between the two heavy chains. Accordingly, the F(ab')2 fragment consists of two Fab' fragments, and the two Fab' fragments are linked to each other by the disulfide bond therebetween.

In the present disclosure, "Fv region" is a fragment of an antibody which includes variable regions of each of a heavy chain and a light chain, but does not include constant regions. In an sdFV, a heavy chain and a light chain are linked by a disulfide bond. In an scFc, single stranded variable regions (Fv) of heavy and light chains are linked via a flexible linker. In an scFv-Fc, an Fc is linked to an scFV. In the minibody, CH3 is linked to an scFV. The diabody includes the scFVs of two molecules.

In the present disclosure, "short-chain antibody (scAb)" is a single polypeptide chain including one variable region of a heavy chain or a light chain constant region in which heavy chain and light chain variable regions are linked by a flexible linker.

In the present disclosure, "domain antibody (dAb)" is an immunologically functional immunoglobulin fragment including only a variable region of a heavy chain or a variable region of a light chain.

In the present disclosure, "bivalent antigen-binding protein" or "bivalent antibody" includes two antigen-binding sites. The two antigen binding sites included in such a bivalent antibody may have the same antigen specificity or may be a bispecific antibody that individually binds to different antigens.

In the present disclosure, "multi-specific antigen-binding protein" or "multi-specific antibody" targets two or more antigens or epitopes.

In the present disclosure, "bispecific" or "dual-specific" antigen-binding protein or antibody is a hybrid antigen-binding protein or antibody having two different antigen-binding sites. These bispecific antibodies are types of multi-specific antigen-binding proteins or multi-specific antibodies and may be produced by various known methods, for example, fusion of hybridomas or linking of Fab' fragments.

In an embodiment, the additional polypeptide includes an additional antibody or a fragment thereof. Additional antibodies include, for example, intact IgG, IgE and IgM, bispecific or multi-specific antibodies (e.g., Zybodies^{®} and the like), single chain Fvs, polypeptide-Fc fusions, Fab, cameloid antibodies, masked antibodies (e.g., Probodies^{®}), small modular immunopharmaceuticals ("SMIPsTM"), single chain or tandem diabodies (TandAb^{®}), VHHs (including those described in the present disclosure without limitation), Anticalins^{®}, Nanobodies^{®}, minibodies, BiTE^{®}, ankyrin repeat proteins or DARPIN^{®}, Avimers^{®}, DART, TCR-like antibodies, Adnectins^{®}, Affilins^{®}, Trans-bodies^{®}, Affibodies ^{®}, TrimerX^{®}, MicroProteins, Fynomers^{®}, Centyrins^{®}, and KALBITOR^{®}. In addition, additional antibodies target PD-1, TIM-3, LAG-3, IDO, A2AR, TGF-beta, CD47, or another protein involved in the immunosuppressive pathway.

In an embodiment, the additional polypeptide includes or consists of part or all of a tumor-associated antigen (TAA) or a tumor-specific antigen (TSA). Non-limiting examples of TSA or TAA antigens include: differentiation antigens such as MART-1/MelanA (MART-I), gp100 (Pmel 17), tyrosinase, TRP-1, and TRP-2, and tumor-specific multilineage antigens such as MAGE- 1, MAGE-3, BAGE, GAGE-1, GAGE-2, and p15; overexpressed embryonic antigens such as CEA; overexpressed oncogenes and mutated tumor-suppressor genes such as p53, Ras, and HER-2/neu; intrinsic tumor antigens resulting from chromosomal translocations, such as BCR-ABL, E2A-PRL, H4-RET, IGH-IGK, and MYL-RAR; and viral antigens such as Epstein-Barr virus antigen EBVA and human papillomavirus (HPV) antigens E6 and E7. Other tumor antigens include TSP-180, MAGE-4, MAGE-5, MAGE-6, RAGE, NY-ESO, erbB, p185erbB2, p180erbB-3, c-met, nm-23H1, PSA, TAG-72, CA 19-9, CA 72-4, CAM 17.1, NuMa, K-ras, beta-catenin, CDK4, Mum-1, p 15, p 16, 43-9F, 5T4, 791Tgp72, alpha-fetoprotein, beta-HCG, BCA225, BTAA, CA 125, CA15-3\CA 27.29\BCAA, CA 195, CA 242, CA-50, CAM43, CD68\P1, CO-029, FGF-5, G250, Ga733\EpCAM, HTgp-175, M344, MA-50, MG7-Ag, MOV18, NB/70K, NY-CO-1, RCAS1, SDCCAG16, TA-90\Mac-2 binding protein\cyclophilin C-associated protein, TAAL6, TAG72, TLP, MUC16 , IL13Rα2, FRα, VEGFR2, Lewis Y, FAP, EphA2, CEACAM5, EGFR, CA6, CA9, GPNMB, EGP1, FOLR1, endothelial receptor, STEAP1, SLC44A4, nectin-4, AGS-16, guanylyl cyclase C, MUC -1, CFC1B, integrin alpha 3 chain (a3b1, laminin receptor chain), and TPS.

In an embodiment, the additional polypeptide includes or consists of part or all of a tumor antigen selected from CD19, CD20, CD22, CD30, CD72, CD180, CD171 (L1CAM), CD123, CD133, CD138, CD37, CD70, CD79a, CD79b, CD56, CD74, CD166, CD71, CLL-1/CLECK12A, ROR1, glypican 3 (GPC3), mesothelin CD33/IL3Ra, c-Met, PSCA, PSMA, glycolipid F77, EGFRvIII, GD-2, MY-ESO-1, and MAGE A3.

In an embodiment, the cancer immunotherapeutic agent may include a fusion protein including a membrane protein and a cytokine that are involved in immunoregulation. For example, the fusion protein may include a membrane protein or a fragment thereof, an Fc region, and a cytokine or a variant thereof.

The term "membrane protein" as used herein refers to a protein that is inserted into a cell membrane consisting of a lipid bilayer or attached to the surface thereof, and that binds to a ligand to transmit costimulatory responses and coinhibitory responses, thereby participating in immune regulation. In an embodiment, the membrane protein may be any one protein belonging to the B7 family, and more specifically, the membrane protein may be CD80 or a fragment thereof. CD80, also known as B7-1, is a transmembrane protein expressed on the surface of T cells, B cells, dendritic cells, and monocytes. CD80 is known to bind to CD28, CTLA4 (CD152), and PD-L1. CD80, CD86, CTLA4, and CD28 are involved in the costimulatory-coinhibitory system. For example, CD80, CD86, CTLA4, and CD28 regulate the activity of T cells and are involved in proliferation, differentiation, and survival.

The term "cytokine" as used herein refers to a category of small proteins with a size of about 5 kDa to about 20 kDa that are important in cell signaling produced by various cells as well as immune cells, which act through cell receptors to regulate the maturation, differentiation, activity and the like of specific immune cell populations, thereby modulating the balance of immune responses. In an embodiment, the cytokine may be an interleukin, more specifically, IL-2 or a variant thereof. IL-2 stimulates the proliferation and differentiation of T cells, and induces the production of cytotoxic T lymphocytes (CTLs) and the differentiation of peripheral blood lymphocytes into cytotoxic cells and lymphokine-activated killer cells (LAK cells). IL-2 is involved in the proliferation and differentiation of B cells, promotes the synthesis of immunoglobulins by B cells, and stimulates the production, proliferation and activation of natural killer cells (NK cells).

In an embodiment, the cancer immunotherapeutic agent may be a fusion protein including CD80 and an IL-2 protein. More specifically, the cancer immunotherapeutic agent may be a fusion protein including a CD80 protein or a fragment thereof and an IL-2 protein or a variant thereof.

The CD80 may be full-length CD80 or a CD80 fragment. CD80 consists of 288 amino acids and specifically, may have the amino acid sequence of SEQ ID NO: 2. The fragment of CD80 may be an extracellular domain of CD80. In an embodiment, the CD80 fragment may be one in which amino acids 1 to 34, which are the signal sequence of CD80, are deleted from the N-terminus. Specifically, the CD80 fragment may be a protein consisting of amino acids 35 to 242 of SEQ ID NO: 2. In an embodiment, the CD80 fragment may have the amino acid sequence of SEQ ID NO: 3.

The IL-2 may be wild-type IL-2 or a variant thereof. The wild-type IL-2 may have the amino acid sequence of SEQ ID NO: 4. The IL-2 variant may have some amino acid substitutions in wild-type IL-2. In an embodiment, the IL-2 variant, which is obtained by amino acid substitution, may have at least one amino acid substitution at positions 38, 42, 45, 61, and 72 in the amino acid sequence of SEQ ID NO: 4.

Specifically, the IL-2 variant may have at least one substitution selected from the group consisting of R38A, F42A, Y45A, E61R, and L72G in the amino acid sequence of SEQ ID NO: 4.

Preferably, in an embodiment, the IL-2 variant may have any one combination of substitutions selected from combinations (a) to (d) in the amino acid sequence of SEQ ID NO: 4:
(a)R38A/F42A;
(b)R38A/F42A/Y45A;
(c)R38A/F42A/E61R; and
(d)R38A/F42A/L72G.

In an embodiment, the IL-2 variant may have the amino acid sequences of SEQ ID NOs: 5 to 8.

The CD80 or a fragment thereof and the IL-2 or a variant thereof may be linked to each other via a linker or carrier. In the present disclosure, "linker" and "carrier" may be used interchangeably. The linker links two proteins. In an embodiment, the linker may include 1 to 50 amino acids, albumin or a fragment thereof, an Fc domain of an immunoglobulin, or the like. In this regard, the Fc domain of the immunoglobulin refers to a protein that contains heavy chain constant region 2 (CH2) and heavy chain constant region 3 (CH3) of an immunoglobulin, and does not contain heavy and light chain variable regions and light chain constant region 1 (CH1) of an immunoglobulin. The immunoglobulin may be IgG, IgA, IgE, IgD, or IgM, and may preferably be IgG4. In this regard, an Fc domain of wild-type immunoglobulin G4 may have the amino acid sequence of SEQ ID NO: 9.

Specifically, the fusion protein may have a structure in which, using an Fc domain as a linker (or carrier), a CD80 protein and an IL-2 protein, or IL-2 and CD80 are linked to the N-terminus and the C-terminus of the linker, respectively. The linkage between the N-terminus or C-terminus of the Fc domain and CD-80 or IL-2 may optionally be achieved by a linker peptide.

In an embodiment, the fusion protein may consist of Structural Formula (I) or (II):
N'-X-[linker (1)]n-Fc domain-[linker (2)]m-Y-C' (I); and
N'-Y-[linker (1)]n-Fc domain-[linker (2)]m-X-C' (II),
wherein, in Structural Formulae (I) and (II),
N' is the N-terminus of the fusion protein,
C' is the C-terminus of the fusion protein,
X is a CD80 protein or a fragment thereof,
Y is an IL-2 protein or a variant thereof,
the linkers (1) and (2) are peptide linkers, and
n and m are each independently 0 or 1.

Preferably, the fusion protein may consist of Structural Formula I. The CD80 protein or a fragment thereof and the IL-2 protein or a variant thereof are as described above.

A peptide linker (1) may be included between the CD80 protein and the Fc domain. The peptide linker (1) may consist of 5 to 80 consecutive amino acids, 20 to 60 consecutive amino acids, or 25 to 50 consecutive amino acids, or 30 to 40 amino acids. In an embodiment, the peptide linker (1) may consist of 30 amino acids. In addition, the peptide linker (1) may contain at least one cysteine. Specifically, the peptide linker (1) may contain one, two, or three cysteines. In addition, the peptide linker (1) may be derived from the hinge of an immunoglobulin. In an embodiment, the peptide linker (1) may be a peptide linker consisting of the amino acid sequence of SEQ ID NO: 10.

The peptide linker (2) may consist of 1 to 50 consecutive amino acids, or 3 to 30 consecutive amino acids, or 5 to 15 amino acids. In an embodiment, the peptide linker (2) may be (G4S)n (wherein n is an integer from 1 to 10). In (G4S)n, n may be 1, 2, 3, 4, 5, 6, 7, 8, 9, or 10. In an embodiment, the peptide linker (2) may be a peptide linker consisting of the amino acid sequence of SEQ ID NO: 11.

In an embodiment, the fusion protein may have at least 90 %, 91 %, 92 %, 93 %, 94 %, 95 %, 96 %, 97 %, 98 %, 99 % or 100 % sequence identity to the amino acid sequences of SEQ ID NOs: 12 to 15. More specifically, the fusion protein may have the amino acid sequences of SEQ ID NOs: 12 to 15.

In an embodiment, the fusion protein may be encoded by a polynucleotide having at least 90 %, 91 %, 92 %, 93 %, 94 %, 95 %, 96 %, 97 %, 98 %, 99 % or 100 % sequence identity to the nucleic acid sequences of SEQ ID NOs: 17 to 20. More specifically, the fusion protein may have the nucleic acid sequences of SEQ ID NOs: 17 to 20.

The polynucleotide encoding the fusion protein may further include a nucleic acid encoding a signal sequence or a leader sequence. The term "signal sequence" as used herein refers to a signal peptide that directs the secretion of a target protein. The signal peptide is cleaved after being translated in a host cell. Specifically, the signal sequence is an amino acid sequence that initiates migration of a protein across the endoplasmic reticulum (ER) membrane. In an embodiment, the signal sequence may have the amino acid sequence of SEQ ID NO: 16.

In an embodiment, two identical fusion proteins may bind to each other to form a homodimer. In this regard, fusion proteins may bind to each other by a disulfide bond via cysteines present in the linker, thereby forming a dimer. More specifically, the fusion proteins constituting a dimer may be proteins having the amino acid sequences of SEQ ID NOs: 12 to 14.

In an embodiment, the second active substance may further include a second anticancer agent. In this regard, the first anticancer agent and the second anticancer agent included in the second active substance may be different from each other.

In an embodiment, the second anticancer agent may be any one selected from the group consisting of a chemotherapeutic agent, a targeted anticancer agent, and a cancer immunotherapeutic agent.

The term "chemotherapeutic agent" as used herein is also referred to as an antineoplastic agent or a cytotoxic agent. The chemotherapeutic agent is a generic term for drugs that exhibit anticancer activity mainly by acting directly on DNA to block DNA replication, transcription and translation processes, or by interfering with the synthesis of nucleic acid precursors in the metabolic pathway and by inhibiting cell division. The antineoplastic agent exhibits cytotoxicity by acting not only on tumor cells but also on normal cells. The chemotherapeutic agent may be used in maintenance therapy. In addition, the term "maintenance therapy" as used herein refers to treatment of cancer with drugs after initial anticancer treatment, and refers to a treatment method performed to prevent or delay cancer recurrence.

Specifically, the chemotherapeutic agent may be any one selected from the group consisting of an alkylating agent, a microtubule inhibitor, an antimetabolite, and a topoisomerase inhibitor. The alkylating agent may be any one selected from the group consisting of mechlorethamine, cyclophosphamide, ifosfamide, melphalan, chlorambucil, thiotepa, altretamine, procarbazine, busulfan, streptozocin, carmustine, lomustine, dacarbazine, cisplatin, carboplatin, and oxaliplatin. The microtubule inhibitor may be any one selected from the group consisting of docetaxel, velban, oncovin, and navelbine. The antimetabolite may be any one selected from the group consisting of 5-fluorouracil, capecitabine, cytarabine, gemcitabine, fludarabine, methotrexate, pemetrexed, and mercaptopurine. The topoisomerase inhibitor may be any one selected from the group consisting of hycamtin, camptosar, vepesid, paclitaxel, blenoxane, adriamycin, etoposide, and cerubidine.

The term "targeted anticancer agent" as used herein is a therapeutic agent that specifically kills cancer cells by blocking signals involved in the growth and development of cancer by targeting specific proteins or specific genetic changes that are frequently present only in cancer cells. The targeted anticancer agent is classified into monoclonal antibodies that react outside cells, and small molecule substances that act inside cells. Monoclonal antibodies are anticancer agents that block cancer cell-inducing signals transmitted to the outside of cells, and act on initiation signals related to proliferation, death, and the like, while small molecule substances act on complex signal transduction occurring inside the cells.

Specifically, the targeted proteins may be epidermal growth factor receptor (EGFR), vascular endothelial growth factor receptor (VEGFR), CD20, CD38, RNAK-L, BTK, Bcr-abl, PDGFR/FGFR family, MEK/RAF, HER2/Neu, ubiquitin, JAK, MAP2K, ALK, PARP, tumor growth factor β receptor (TGFβR), proteasome, Bcl-2, C-Met, VR1, VR2, VR3, c-kit, AXL, RET, Braf, DNA methyltransferase (DNMT), CDK4/6, STING, and the like.

The targeted anticancer agent may be any one selected from the group consisting of olaratumab, erlotinib, panitumumab, trastuzumab, trastuzumab emtansine, pertuzumab, cetuximab, rituximab, bevacizumab, axitinib, lenvatinib, ramucirumab, aflibercept, obinutuzumab, daratumumab, denosumab, ibrutinib, dasatinib, radotinib, nilotinib, imatinib, bosutinib, galunisertib, vactosertib, nintedanib, sunitinib, sorafenib, cabozantinib, regorafenib, masitinib, semaxanib, ceritinib, tivozanib, brigatinib, vandetanib, pazopanib, trametinib, temsirolimus, dabrafenib, dacomitinib, afatinib, lapatinib, neratinib, lenalidomide, osimertinib, ixazomib, olmutinib, everolimus, ruxolitinib, lestaurtinib, pacritinib, cobimetinib, selumetinib, binimetinib, bortezomib, alectinib, crizotinib, venetoclax, bemcentinib, gilteritinib, selpercatinib, pralsetinib, vemurafenib, olaparib, talazoparib, niraparib, rucaparib, azacitidine, decitabine, guadecitabine, gefitinib, abemaciclib, ribociclib, palbociclib, and DMXAA.

The term "epidermal growth factor receptor (EGFR)" as used herein is a cell membrane receptor that regulates cell growth, division, survival and death. In various cancers, the expression of EGFR is increased in tumor tissues. It is known that tumor tissues with the increased EGFR are invasive, metastatic, and highly resistant to anticancer agents. The EGFR inhibitor may be a substance that inhibits the EGFR, and in an embodiment, may be cetuximab, trastuzumab, pertuzumab, gefitinib, erlotinib, or panitumumab.

The term "vascular endothelial growth factor receptor (VEGFR)" as used herein is a cell membrane receptor of a vascular endothelial growth factor that induces angiogenesis, and a VEGFR inhibitor inhibits the angiogenesis to suppress tumor growth and metastasis. In an embodiment, the VEGF inhibitor or VEGFR inhibitor may be axitinib, lenvatinib, bevacizumab, ramucirumab, or aflibercept.

The term "CD20 (B lymphocyte antigen CD20)" as used herein is a protein expressed on the surface of B cells and is used as a target protein for the treatment of B cell lymphoma. The CD20-targeting inhibitor may be rituximab or obinutuzumab.

The term "cluster of differentiation 38 (CD38)" as used herein refers to a protein that regulates cell growth and death while acting as a signal transduction receptor in immune cells, and an inhibitor targeting this may be daratumumab.

The term "receptor activator of nuclear factor kappa-B ligand (RNAK-L)" as used herein is a RANK receptor expressed on the surface of osteoclasts, which causes bone destruction when activated by binding to a ligand thereof. The RANK-L inhibitor is mainly used for cancer patients suffering from bone metastases or osteoporosis, and may specifically be denosumab.

The term "Bruton's tyrosine kinase (BTK)" as used herein is an enzyme that is involved in the proliferation of B cells and may develop into hematologic malignancy when overexpressed. In an embodiment, the BTK-targeting inhibitor may be ibrutinib.

The term "Bcr-abl" as used herein is a fusion protein that is highly expressed in chronic myelogenous leukemia patients, and is known to induce abnormal proliferation of blood cells. Specifically, an inhibitor against the protein may be dasatinib, nilotinib, imatinib, or bosutinib.

The term "tumor growth factor β receptor (TGFβR)" as used herein is a cell membrane receptor of a tumor growth factor that regulates the growth, migration, differentiation, death and the like of epithelial cells and hematopoietic cells. The TGFβR-targeting inhibitor may be, but is not limited to, galunisertib or vactosertib.

The term "platelet derived growth factor receptor (PDGFR)" as used herein is a cell membrane receptor for PDGF that is frequently expressed in cancer cells, and is known to be involved in angiogenesis to regulate cancer growth, cancer metastasis, and drug resistance. Fibroblast growth factor receptor (FGFR) is a receptor of fibroblast growth factor (FGF), which regulates various biological processes including cell growth, differentiation, migration, and the like. The FGFR gene is easily mutated, and these variants are commonly observed in breast cancer, uterine cancer, ovarian cancer, cervical cancer, and the like. An inhibitor targeting PDGFR or FGFR may be nintedanib, sunitinib, sorafenib, cabozantinib, lenvatinib, regorafenib, masitinib, semaxanib, tivozanib, vandetanib, axitinib, or pazopanib.

The term "MEK/RAF" as used herein is an intracellular signaling mediator involved in cell proliferation, cell cycle regulation, cell survival, angiogenesis, cell migration, and the like, and is overactivated in cancer cells. An inhibitor targeting MEK/RAF may be trametinib or dabrafenib.

The term "human epidermal growth factor receptor 2 (HER-2/neu)" as used herein regulates cell proliferation through activation of PI3K/AkT. It is known that HER-2/neu is overexpressed in metastatic breast cancer, ovarian cancer, and the like, and induces resistance to anticancer agents. A Her2/neu-targeting anticancer agent may be trastuzumab, afatinib, lapatinib, or neratinib.

The term "ubiquitin" as used herein maintains cell homeostasis by binding to other proteins and inducing proteolysis (ubiquitin-proteasome system, UPS) by proteasome, which is a proteolytic enzyme. Abnormal expression or activity of the UPS is observed in various tumors, and ubiquitin inhibitors exhibit anticancer activity. Specifically, an inhibitor targeting ubiquitin or proteasome may be lenalidomide or ixazomib.

The term "Janus kinase (JAK)" as used herein is an upstream protein of STAT, which is a transcription factor that regulates cell proliferation, cell survival, cell migration, and immune responses. A JAK inhibitor is known to reduce cell proliferation and induce apoptosis, by inhibiting the activity of STAT. The JAK-targeting inhibitor may be ruxolitinib, lestaurtinib, or pacritinib.

The term "mitogen-activated protein kinase (MAP2K)" as used herein is an intracellular signaling mediator that is involved in cell proliferation, cell cycle regulation, cell survival, angiogenesis, cell migration and the like by phosphorylating MAPK, and is overactivated in cancer cells. An MAP2K-targeting inhibitor may be cobimetinib, selumetinib, trametinib, or binimetinib.

The term "anaplastic lymphoma kinase (ALK)" as used herein is a signaling mediator that promotes cell proliferation, cell migration, and angiogenesis and inhibits cell death, and is overactivated in various cancer tissues. An ALK-targeting inhibitor may be alectinib or crizotinib.

The term "Bcl-2" as used herein is a protein that inhibits cell death, and is overexpressed or overactivated in various cancer tissues. An inhibitor targeting Bcl-2 may be venetoclax.

The term "C-Met" as used herein is a receptor of hepatocyte growth factor (HGF), which activates signal transduction related to cell growth, formation, motility, survival, angiogenesis, and the like. A C-Met-targeting anticancer agent may be crizotinib or cabozantinib.

The term "vanilloid receptor (VR)" as used herein is also known as transient receptor potential vanilloid (TRPV), and exists in the form of VR1, VR2, VR3, VR4, VR5, and VR6. VR is known to regulate the proliferation, death, migration, invasion and angiogenesis of cancer cells at each stage in the cancer progression process.

The term "c-kit" as used herein is also known as CD117, and induces signal transduction that activates cell survival, proliferation and differentiation. c-kit is a proto-oncogene, and overexpression or mutation of the gene is related to the onset of cancer.

The term "AXL (Yyrosin-protein kinase receptor UFO)" as used herein is a tyrosine kinase receptor present on the surface of a cell and mediates signal transduction involved in cell proliferation and survival. AXL is known to be involved in anticancer drug resistance in anticancer treatment. In an embodiment, an AXL-targeting anticancer agent may be bemcentinib or gilteritinib.

The term "rearranged during transfection (RET)" as used herein is a receptor that mediates signals involved in cell proliferation, cell death, and survival, and mutations in RET are known to be involved in cancer development. A RET-targeting inhibitor may be, but is not limited to, selpercatinib or pralsetinib.

The term "Braf" as used herein is a MAPK signaling mediator involved in cell proliferation, cell cycle regulation, cell survival, angiogenesis, cell migration, and the like, and genetic mutations thereof are observed in cancer cells. An inhibitor targeting Braf may be vemurafenib.

The term "poly[ADP-ribose]polymerase (PARP)" as used herein is a protein that recognizes damaged DNA in the nucleus and is activated, and then activates a DNA repair-related protein. An PARP-targeting inhibitor suppresses the proliferation of cancer cells by inhibiting DNA repair of cancer cells. In an embodiment, the PARP-targeting inhibitor may be olaparib, talazoparib, niraparib, or rucaparib.

The term "DNA methyltransferase (DNMT)" as used herein is an enzyme that attaches a methyl group to the histone protein surrounding DNA, and gene expression is inhibited through the above process. An DMNT-targeting inhibitor exhibits anticancer activity by inhibiting hypermethylation of cancer suppressor genes and inducing normal expression of the cancer suppressor genes. In an embodiment, the DNMT-targeting inhibitor may be azacitidine, decitabine, or guadecitabine.

The term "cyclin dependent kinase (CDK) 4/6" as used herein is a protein that regulates the cell cycle and promotes cell growth, and is overactivated in the development and progression stages of various malignant tumors. An CDK4/6-targeting inhibitor exhibits anticancer activity by inhibiting the cell cycle of cancer cells, inhibiting cell proliferation and inducing apoptosis. The CDK4/6-targeting inhibitor may be abemaciclib or palbociclib.

The term "stimulator of interferon genes (STING)" as used herein is an in vivo sensor that recognizes DNA fragments derived from cancer cells, and activates immune cells in the body such as dendritic cells by stimulating interferon genes. An agonist of the STING exhibits an immune enhancing effect and a cancer angiogenesis inhibitory effect. For example, the STING agonist may be CDNs, SB11285, DMXAA, or the like.

The term "antibody therapeutic agent" as used herein is a therapeutic agent that exhibits anticancer effect by using an antibody that recognizes a specific protein of cancer cells as an antigen. The antibody therapeutic agent may be any one selected from the group consisting of cetuximab, trastuzumab, trastuzumab emtansine, rituximab, ibritumomab, tositumomab, brentuximab, ofatumumab, obinutuzumab, necitumumab, bevacizumab, ramucirumab, nivolumab, pembrolizumab, atezolizumab, durvalumab, and ipilimumab, but the present disclosure is not limited thereto.

The term "cancer immunotherapeutic agent" as used herein is a substance that inhibits the activity of an immune checkpoint protein that inhibits the differentiation, proliferation and activity of immune cells, and is known to eradicate cancer cells by preventing the cancer cells from exerting the function of evading the immune system. The cancer immunotherapeutic agent may be an antibody against any one selected from the group consisting of 2B4, 4-1BB (CD137), AaR, B7-H3, B7-H4, BAFFR, BTLA, CD2, CD7, CD27, CD28, CD30, CD40, CD80, CD83 ligand, CD86, CD160, CD200, CDS, CEACAM, CTLA-4, GITR, HVEM, ICAM-1, KIR, LAG-3, LAIR1, LFA-1 (CD 11 a/CD 18), LIGHT, NKG2C, NKp80, OX40, PD-1, PD-L1, PD-L2, SLAMF7, TGFRp, TIGIT, Tim3, and VISTA. More specifically, the cancer immunotherapeutic agent may be any one selected from the group consisting of an anti-CTLA-4 antibody, an anti-PD-1 antibody, an anti-PD-L1 antibody, an anti-PD-L2 antibody, an anti-B7-H4 antibody, an anti-HVEM antibody, an anti-TIM3 antibody, an anti-GAL9 antibody, an anti-LAG3 antibody, an anti-VISTA antibody, an anti-KIR antibody, an anti-BTLA antibody, and an anti-TIGIT antibody, but the present disclosure is not limited thereto. In an embodiment, the cancer immunotherapeutic agent may be any one selected from the group consisting of atezolizumab, avelumab, durvalumab, nivolumab, pembrolizumab, abagovomab, adecatumumab, afutuzumab, alemtuzumab, anatumomab mafenatox, mepolizumab, blinatumomab, BMS-936559, catumaxomab, cemiplimab, epacadostat, epratuzumab, indoximod, inotuzumab, inotuzumab ozogamicin, intelumumab, ipilimumab, isatuximab, lambrolizumab, MED 14736, MPDL3280A, obinutuzumab, ocaratuzumab, ofatumumab, olaratumab, pidilizumab, rituximab, ticilimumab, samalizumab, and tremelimumab, but the present disclosure is not limited thereto.

In addition, the cancer immunotherapeutic agent may be a fusion protein including a membrane protein and a cytokine, and specifically, may be a fusion protein including a C80 protein or a fragment thereof and an IL-2 protein or a variant thereof. The fusion protein including CD80 and IL-2 is as described above.

The term "antibody drug conjugate (ADC)" as used herein is a therapeutic agent that exhibits a high anticancer effect through targeted delivery by chemical binding between an antibody and a cytotoxic drug. The ADC may be gemtuzumab-ozogamicin, brentuximab-vedotin, trastuzumab-emtansine, inotuzumab-ozogamicin, eribulin-mesylate, and the like.

The anticancer agent may be any one selected from the group consisting of cisplatin, oxaliplatin, ALTIMA, axitinib (VR1,2,3, PDGFR, c-kit), galunisertib (TGFβRI), lenvatinib (VR1,2,3), ramucirumab (VR2), cabozatinib (c-Met, VR2, AXL, RET), olaparib (PARP), guadecitabine (DNMT), docetaxel, paclitaxel, pemetrexed, vemurafenib (Braf), abemaciclib (CDK4/6), cetuximab (EGFR), durvalumab (PD-L1), trastuzumab (Her2), DMXAA, and keytruda (PD-1).

The *Lactobacillus plantarum* GB104 strain may be used in combination with the anticancer agent and an anticancer vaccine. In this regard, the *Lactobacillus plantarum* GB104 strain and the anticancer agent are as described above.

The term "anticancer vaccine" as used herein is active immunotherapy by which cancer cells are removed by enhancing the immune function in vivo through activation of the immune system via administration of a tumor-specific antigen (TSA) of cancer cells to cancer patients. Anticancer vaccines include DNA vaccines, peptide vaccines, cell vaccines, and the like, depending on the type of antigen and an antigen delivery method. Currently, cell vaccines and DNA vaccines developed by introducing antigens are typically being developed.

In an embodiment, the *Lactobacillus plantarum* GB104 strain may be used in combination with two or more different anticancer agents (e.g., a first anticancer agent and a second anticancer agent). For example, the two anticancer agents (e.g., a first anticancer agent and a second anticancer agent) may be: a chemotherapeutic agent and a targeted anticancer agent; a chemotherapeutic agent and an anticancer virus; a targeted anticancer agent and an antibody therapeutic agent; a chemotherapeutic agent and a cellular therapeutic agent; and a chemotherapeutic agent and a cancer immunotherapeutic agent. Also, the two anticancer agents may be: a targeted anticancer agent and an anticancer virus; a targeted anticancer agent and an antibody therapeutic agent; a targeted anticancer agent and a cellular therapeutic agent; and a targeted anticancer agent and a cancer immunotherapeutic agent. Also, the two anticancer agents may be: an anticancer virus and an antibody therapeutic agent; an anticancer virus and a cellular therapeutic agent; and an anticancer virus and a cancer immunotherapeutic agent. Also, the two anticancer agents may be: an antibody therapeutic agent and a cellular therapeutic agent; and an antibody therapeutic agent and a cancer immunotherapeutic agent. In an embodiment of the present disclosure, the two anticancer agents may be: a cancer immunotherapeutic agent and a chemotherapeutic agent; a cancer immunotherapeutic agent and a targeted anticancer agent; or a cancer immunotherapeutic agent and an antibody therapeutic agent.

In an embodiment, the first anticancer agent co-administered with the *Lactobacillus plantarum* GB104 strain may be a fusion protein including a CD80 protein or a fragment thereof and an IL-2 protein or a variant thereof, and the second anticancer agent co-administered with the *Lactobacillus plantarum* GB104 strain may be one or more selected from the group consisting of 5-fluorouracil, anti-PD-1, bevacizumab, cetuximab, and regorafenib.

In an embodiment, the first anticancer agent and the second anticancer agent may be co-administered simultaneously, sequentially, or in reverse order.

The term "combined therapy," "co-administration," or "in combination," as used herein, refers to any form of simultaneous or concurrent treatment using at least two separate therapeutic agents. Ingredients of the combined therapy may be administered simultaneously, sequentially, or in any order. The ingredients may be appropriately administered at different dosages, different frequencies of administration, or via different routes.

Specifically, for the co-administration, the first anticancer agent and the second anticancer agent may be administered simultaneously, or the first anticancer agent may be administered, followed by administration of the second anticancer agent. The combined therapy according to the present disclosure may be defined as being capable of providing a synergistic effect if the efficacy measured through, for example, the degree of response, the rate of response, the disease progression duration, or the survival period is therapeutically superior to the efficacy obtained by administering one or the remainder of the ingredients of the combed therapy at a normal dose. For example, if the efficacy thereof is therapeutically superior to the efficacy obtained using each of the ingredients alone, the efficacy of the combined therapy is synergistic. In particular, it is considered that there is a synergistic effect if normal doses of the first anticancer agent and the second anticancer agent can be reduced while the side effects are reduced/reduced or small, compared with those occurring when each ingredient is used at a normal dose, without adversely affecting one or more of the degree of response, the rate of response, the disease progression duration, and survival data, particularly the response duration.

The term "administered simultaneously" as used herein is not particularly limited and means that the ingredients of the combined therapy are substantially administered at the same time, e.g., as a mixture or in immediate subsequent order.

The term "administered sequentially" as used herein is not particularly limited and means that the ingredients of the combined therapy are not administered simultaneously, but are administered one by one or in clusters with a specific time interval between administrations. The time interval may be the same or different between the respective administrations of the ingredients of the combined therapy and may be selected, for example, from the range of 2 minutes to 96 hours, 1 day to 7 days, or one, two or three weeks. Generally, the time interval between administrations may be in the range of several minutes to several hours, such as in the range of 2 minutes to 72 hours, 30 minutes to 24 hours, or 1 hour to 12 hours. Further examples thereof include time intervals in the range of 24 hours to 96 hours, 12 hours to 36 hours, 8 hours to 24 hours, and 6 hours to 12 hours.

In an embodiment, the first anticancer agent and the second anticancer agent may be administered via independent routes. Each active ingredient may be independently administered by those of ordinary skill in the art at a suitable dose using a suitable administration method. Specifically, the first anticancer agent and the second anticancer agent may be administered intratumorally, intraarterially, intravenously, intravascularly, intrapleurally, intraperitoneally, intratracheally, intrathecally, intramuscularly, endoscopically, intralesionally, transdermally, subcutaneously, regionally, stereotactically, orally, or by direct injection or perfusion. Specifically, the first anticancer agent and the second anticancer agent may be administered orally, intravenously, or subcutaneously. More specifically, the first anticancer agent may be administered subcutaneously or intravenously, and the second anticancer agent may be administered intraperitoneally or intravenously, but the present disclosure is not limited thereto.

In an embodiment, the second active substance may include the first anticancer agent or the first and second anticancer agents alone as an active ingredient, or may include one or more pharmaceutically acceptable carriers, excipients or diluents.

In an embodiment, the anticancer agent may be prepared in a separate formulation to be administered in combination with the composition of the present disclosure. The anticancer agent may be prepared as an injectable agent including the same, preferably as a formulation administered intratumorally, intraarterially, intravenously, intravascularly, intrapleurally, intraperitoneally, intratracheally, intrathecally, intramuscularly, endoscopically, intralesionally, transdermally, subcutaneously, regionally, stereotactically, orally, or by direct injection or perfusion, but the present disclosure is not limited thereto.

The term "including, as an active ingredient" as used herein refers to the addition of a strain of the genus *Lactobacillus,* a lysate of the strain, a culture broth of the strain, or an extract of the culture broth, and is intended to include the addition of various ingredients as sub-ingredients for drug delivery, stabilization, and the like and formulation thereof in various forms.

In an embodiment, the first active substance and the second active substance may be co-administered simultaneously, sequentially, or in reverse order.

The "strain," "first active substance," "second active substance," "first anticancer agent," "second anticancer agent," and "co-administration" are as described above.

Specifically, the co-administration may be simultaneous administration of the *Lactobacillus plantarum* strain and the anticancer agent, or administration of the *Lactobacillus plantarum* strain, followed by administration of the anticancer agent. In this regard, the anticancer agent may include a first anticancer agent and a second anticancer agent which are different from each other. More specifically, the first anticancer agent may be a cancer immunotherapeutic agent. The combined therapy according to the present disclosure may be defined as being capable of providing a synergistic effect if the efficacy measured through, for example, the degree of response, the rate of response, the disease progression duration, or the survival period is therapeutically superior to the efficacy obtained by administering one or the remainder of the ingredients of the combed therapy at a normal dose. For example, if the efficacy thereof is therapeutically superior to the efficacy obtained using each of the ingredients, the efficacy of the combined therapy is synergistic. In particular, it is considered that there is a synergistic effect if normal doses of the *Lactobacillus plantarum* strain and the anticancer agent can be reduced while the side effects are reduced/reduced or small, compared with those occurring when each ingredient is used at a normal dose, without adversely affecting one or more of the degree of response, the rate of response, the disease progression duration, and survival data, particularly the response duration.

In an embodiment, the synergistic effect of the co-administration may be reducing the dose or number of administrations of an existing anticancer agent. Specifically, when the *Lactobacillus plantarum* GB104 strain is administered in combination with a cancer immunotherapeutic agent (e.g., a fusion protein including a CD80 protein or a fragment thereof and an IL-2 protein or a variant thereof), the same or similar anticancer activity may be exhibited at a lower dose or fewer administrations compared with the dose when the cancer immunotherapeutic agent is administered alone. In an embodiment, the anticancer activity in the case of administration intervals of 1 day upon administration of the cancer immunotherapeutic agent alone was the same as or similar to that in the case of administration intervals of 5 days to 10 days (e.g., 7 days) upon co-administration of the *Lactobacillus plantarum* GB104 strain and the cancer immunotherapeutic agent. Therefore, co-administration of *Lactobacillus plantarum* GB104 has a synergistic effect that can reduce the typical dose and frequency of a cancer immunotherapeutic agent.

In an embodiment, when the *Lactobacillus plantarum* GB104 strain is administered in combination with a cancer immunotherapeutic agent (e.g., a fusion protein including a CD80 protein or a fragment thereof and an IL-2 protein or a variant thereof) and a chemotherapeutic agent (e.g., 5-FU), the same or similar anticancer activity may be exhibited at a lower dose or fewer administrations compared with the dose when the cancer immunotherapeutic agent or the chemotherapeutic agent is administered alone. In an embodiment, the anticancer activity in the case of administration intervals of 1 day upon administration of the cancer immunotherapeutic agent or the chemotherapeutic agent alone was the same as or similar to that in the case of administration intervals of 5 days to 10 days (e.g., 7 days) upon co-administration of the *Lactobacillus plantarum* GB104 strain, the cancer immunotherapeutic agent, and the chemotherapeutic agent. Therefore, co-administration of *Lactobacillus plantarum* GB104 has a synergistic effect that can reduce the typical dose and frequency of a cancer immunotherapeutic agent and/or a chemotherapeutic agent.

In an embodiment, the first active substance and the second active substance may be administered via independent routes. Each active ingredient may be independently administered by those of ordinary skill in the art at a suitable dose using a suitable administration method. Specifically, the *Lactobacillus plantarum* GB104 strain and the anticancer agent may be administered intratumorally, intraarterially, intravenously, intravascularly, intrapleurally, intraperitoneally, intratracheally, intrathecally, intramuscularly, endoscopically, intralesionally, transdermally, subcutaneously, regionally, stereotactically, orally, or by direct injection or perfusion. Specifically, the first active substance and the second active substance may be administered orally, intravenously, or subcutaneously. More specifically, the first active substance may be administered orally, and the second active substance may be administered intravenously or subcutaneously, but the present disclosure is not limited thereto.

In an embodiment, the first active substance and the second active substance may each be administered to a subject at intervals of 1 day to 10 days. More specifically, the first active substance may be administered to a subject at intervals of 1 day to 3 days, and the second active substance may be administered to a subject at intervals of 5 days to 10 days. In an embodiment, the first active substance was administered to a subject at intervals of 1 day, and the second active substance was administered to a subject at intervals of 7 days.

Another aspect provides a pharmaceutical composition for preventing or treating cancer, which: includes, as active ingredients, a first active substance including one or more selected from the group consisting of a *Lactobacillus plantarum* strain, a culture of the strain, a lysate of the strain, or an extract of the strain, the culture, and the lysate, and a second active substance including a cancer immunotherapeutic agent as a first anticancer agent; or includes the first active substance as an active ingredient and is administered in combination with the second active substance.

The "strain," "first active substance," "anticancer agent," "second active substance," "anticancer activity," and "co-administration" are as described above.

In an embodiment, the pharmaceutical composition may be administered to mammals, including humans, via various routes. The administration method may be any commonly used method, for example, oral, dermal, intravenous, intramuscular or subcutaneous administration, and may preferably be oral administration.

In an embodiment, the composition may include a first oral formulation including the first active substance and a second oral formulation including the second active substance, and the first and second oral formulations may be administered orally.

The term "prevention" as used herein may refer to any action that suppresses the disease condition of a subject or delays the onset thereof via administration of the pharmaceutical composition according to an aspect.

The term "treatment" as used herein may refer to any action that ameliorates or beneficially alters the symptoms of the disease condition of a subject via administration of the pharmaceutical composition according to an aspect.

The composition according to an embodiment may include the *Lactobacillus plantarum* strain in an amount of 0.001 wt% to 80 wt% with respect to a total weight of the composition. In addition, the dose of the *Lactobacillus plantarum* strain may range from 0.01 mg to 10,000 mg, 0.1 mg to 1000 mg, 1 mg to 100 mg, 0.01 mg to 1000 mg, 0.01 mg to 100 mg, 0.01 mg to 10 mg, or 0.01 mg to 1 mg. The strain may be included in the composition in a therapeutically effective amount or at a nutritionally effective concentration. For example, the strain may be included in an amount of 10³ CFU/g to 10¹⁶ CFU/g, 10³ CFU/g to 10¹⁵ CFU/g, 10³ CFU/g to 10¹⁴ CFU/g, 10³ CFU/g to 10¹³ CFU/g, 10³ CFU/g to 10¹² CFU/g, 10⁴ CFU/g to 10¹⁶ CFU/g, 10⁴ CFU/g to 10¹⁵ CFU/g, 10⁴ CFU/g to 10¹⁴ CFU/g, 10⁴ CFU/g to 10¹³ CFU/g, 10⁴ CFU/g to 10¹² CFU/g, 10⁵ CFU/g to 10¹⁶ CFU/g, 10⁵ CFU/g to 10¹⁵ CFU/g, 10⁵ CFU/g to 10¹⁴ CFU/g, 10⁵ CFU/g to 10¹³ CFU/g, 10⁵ CFU/g to 10¹² CFU/g, 10⁶ CFU/g to 10¹³ CFU/g, 10⁶ CFU/g to 10¹² CFU/g, 10⁷ CFU/g to 10¹³ CFU/g, 10⁷ CFU/g to 10¹² CFU/g, 10⁸ CFU/g to 10¹³ CFU/g, or 10⁸ CFU/g to 10¹² CFU/g, or may be included in the composition as a culture of an equal number of viable or non-viable bacteria. Specifically, for adult patients, 1 x 10³ CFU/g to 1 x 10¹⁶ CFU/g of live or dead bacteria may be administered in single or multiple doses. A dose may vary depending on various factors such as formulation method, administration method, age, body weight and gender of a patient, pathological conditions, diet, administration time, administration route, excretion rate, response sensitivity, and the dose may be appropriately adjusted by those of ordinary skill in the art in consideration of these factors. The pharmaceutical composition may be administered once or administered two or more times to the extent that adverse effects are clinically acceptable, and may be administered to one site or two or more sites. For non-human animals, the pharmaceutical composition may be administered at the same dose as that for humans per kg (body weight), or may be administered, for example, in an amount obtained by converting the dose on the basis of a volume ratio (e.g., average value) of organs (heart or the like) between a target animal and humans. Possible routes of administration may include oral, sublingual, parenteral (e.g., subcutaneous, intramuscular, intraarterial, intraperitoneal, intrathecal, or intravenous), rectal, topical (including transdermal), inhalation, and injection, or insertion of an implantable device or substance. Examples of animals to be treated according to an embodiment include humans and other target mammals, and specific examples thereof include humans, monkeys, mice, rats, rabbits, sheep, cows, dogs, horses, pigs, and the like. In an embodiment, the composition includes a killed dry strain, and may be administered in an amount of 1 g to 10 g, 0.5 g to 1.5 g, 2.5 g to 3.5 g, or 4.5 g to 5.5 g once to 3 times a day.

The term "therapeutically effective amount" as used herein refers to the amount of an anticancer agent for the methods and uses of the present disclosure or a pharmaceutical composition including the anticancer agent for the methods and uses of the present disclosure, which results in biological or medical responses or desired therapeutic effects in patients that researchers, physicians, or other clinicians seek to achieve. The therapeutically effective amount of the anticancer agent may vary depending on factors such as the disease condition, age, gender and body weight of a subject, and the ability of the anticancer agent to induce a desired response in the subject. A therapeutically effective amount also refers to such an amount at which the therapeutic benefit exceeds any toxicity or harmful effects.

The pharmaceutical composition according to an embodiment may include a pharmaceutically acceptable carrier and/or an additive. For example, the pharmaceutical composition may include sterile water, physiological saline, common buffers (phosphoric acid, citric acid, other organic acids, and the like), stabilizers, salts, antioxidants (ascorbic acid and the like), surfactants, suspending agents, tonicity agents, preservatives, or the like. For topical administration, the pharmaceutical composition may be combined with organic substances such as biopolymers, or inorganic substances such as hydroxyapatite, specifically, collagen matrices, polylactic acid polymers or copolymers, polyethylene glycol polymers or copolymers, and chemical derivatives thereof.

When the pharmaceutical composition according to an embodiment is prepared as a formulation suitable for injection, *Lactobacillus* sp. cells may be dissolved or dispersed in a pharmaceutically acceptable carrier, or frozen in such a solution state that they are dissolved or dispersed.

The pharmaceutical composition according to an embodiment, if necessary, depending on the administration method or dosage form, may appropriately include suspending agents, solubilizing agents, stabilizers, isotonic agents, preservatives, anti-adsorption agents, surfactants, diluents, disintegrants, pH adjusting agents, analgesics, buffering agents, reducing agents, and antioxidants. Pharmaceutically acceptable carriers and formulations suitable for the present disclosure, including those exemplified above, are described in detail in the document [Remington's Pharmaceutical Sciences, 19th ed., 1995]. The pharmaceutical composition according to an embodiment may be formulated in a unit dosage form or by inserting into a multidose container, by using a pharmaceutically acceptable carrier and/or excipient according to a method that can be easily performed by one of ordinary skill in the art to which the present disclosure pertains. The dosage form may be in the form of a solution, suspension or emulsion in an oil or aqueous medium, or in the form of a powder, granule, tablet or capsule.

The pharmaceutical composition may be administered in a pharmaceutically effective amount. The term "pharmaceutically effective amount" as used herein refers to an amount sufficient to treat a disease with a reasonable benefit/risk ratio applicable to medical treatment, and the effective dose level may be determined depending on the type and severity of a patient's disease, drug activity, sensitivity to the drug, time of administration, route of administration and excretion rate, duration of treatment, drugs used concurrently, and other factors well known in the medical field. The composition of the present disclosure may be administered as an individual therapeutic agent or in combination with other therapeutic agents, may be administered sequentially or simultaneously with conventional therapeutic agents, and may be administered as a single dose or multiple doses. Considering all of these factors, it is important to administer the minimum effective amount that achieves maximum efficacy without any side effects, which can be readily determined by one of ordinary skill in the art.

In an embodiment, the anticancer activity of the first active substance may be more effective when administered in combination with the second active substance.

In an embodiment, when the *Lactobacillus plantarum* GB104 strain and 5-fluorouracil, which is a chemotherapeutic agent, were co-administered to a tumor animal model transplanted with the mouse colorectal cancer cell line MC-38, a synergistic anticancer effect was shown, compared with administration of the *Lactobacillus plantarum* GB104 strain alone. Specifically, the effect of inhibiting the growth and progression of colorectal cancer tumors was confirmed. On average, compared with a control, the experimental group co-administered with GB104 and the chemotherapeutic agent exhibited an effect of reducing the tumor volume by 10 % to 90 %.

In an embodiment, co-administration of the *Lactobacillus plantarum* GB104 strain and the chemotherapeutic agent may exhibit an activity that reduces the colorectal cancer tumor volume by 90 % or less, 80 % or less, 70 % or less, 67 % or less, 10 % to 90 %, 10 % to 80 %, 10 % to 70 %, 10 % to 67 %, 20 % to 90 %, 20 % to 80 %, 20 % to 70 %, 20 % to 67 %, 30 % to 90 %, 30 % to 80 %, 30 % to 70 %, 30 % to 67 %, 40 % to 90 %, 40 % to 80 %, 40 % to 70 %, 40 % to 67 %, 50 % to 90 %, 50 % to 80 %, 50 % to 70 %, 50 % to 67 %, or 60 % to 67 %, with respect to 100% of the colorectal cancer tumor volume of a negative control that was not administered with the strain and the anticancer agent.

In an embodiment, when the *Lactobacillus plantarum* GB104 strain and anti-PD-1, which is a cancer immunotherapeutic agent, were co-administered to a tumor animal model transplanted with the mouse colon cancer cell line MC-38, a synergistic anticancer effect was shown, compared with administration of the *Lactobacillus plantarum* GB104 strain alone. Specifically, the effect of inhibiting the growth and progression of colorectal cancer tumors was confirmed. On average, compared with a control, the experimental group co-administered with GB104 and the cancer immunotherapeutic agent exhibited an effect of reducing the tumor volume by 10 % to 90 %.

In an embodiment, when the *Lactobacillus plantarum* GB104 strain and the cancer immunotherapeutic agent GI-101 or GI-102, which is a fusion protein including a fragment of CD80 and a variant of an IL-2 protein, were co-administered to a tumor animal model transplanted with the mouse colorectal cancer cell line MC-38, a synergistic anticancer effect was shown, compared with administration of the *Lactobacillus plantarum* GB104 strain alone. Specifically, the effect of inhibiting the growth and progression of colorectal cancer tumors was confirmed. On average, compared with a control, the experimental group co-administered with GB104 and the cancer immunotherapeutic agent exhibited an effect of reducing the tumor volume by 10 % to 90 %.

In an embodiment, co-administration of the *Lactobacillus plantarum* GB104 strain and the cancer immunotherapeutic agent may exhibit an activity that reduces the colorectal cancer tumor volume by 90 % or less, 80 % or less, 70 % or less, 67 % or less, 10 % to 90 %, 10 % to 80 %, 10 % to 70 %, 10 % to 67 %, 20 % to 90 %, 20 % to 80 %, 20 % to 70 %, 20 % to 67 %, 30 % to 90 %, 30 % to 80 %, 30 % to 70 %, 30 % to 67 %, 40 % to 90 %, 40 % to 80 %, 40 % to 70 %, 40 % to 67 %, 50 % to 90 %, 50 % to 80 %, 50 % to 70 %, 50 % to 67 %, or 60 % to 67 %, with respect to 100% of the colorectal cancer tumor volume of a negative control that was not administered with the strain and the anticancer agent.

In an embodiment, when the *Lactobacillus plantarum* GB104 strain and cetuximab, bevacizumab, or regorafenib, which are targeted anticancer agents, were co-administered to a tumor animal model transplanted with the mouse colorectal cancer cell line MC-38, a synergistic anticancer effect was shown, compared with administration of the *Lactobacillus plantarum* GB104 strain alone. Specifically, the effect of inhibiting the growth and progression of colorectal cancer tumors was confirmed. On average, compared with a control, the experimental group co-administered with GB104 and the targeted anticancer agent exhibited an effect of reducing the tumor volume by 10 % to 90 %.

In an embodiment, co-administration of the *Lactobacillus plantarum* GB104 strain and the targeted anticancer agent may exhibit an activity that reduces the colorectal cancer tumor volume by 90 % or less, 80 % or less, 70 % or less, 67 % or less, 10 % to 90 %, 10 % to 80 %, 10 % to 70 %, 10 % to 67 %, 20 % to 90 %, 20 % to 80 %, 20 % to 70 %, 20 % to 67 %, 30 % to 90 %, 30 % to 80 %, 30 % to 70 %, 30 % to 67 %, 40 % to 90 %, 40 % to 80 %, 40 % to 70 %, 40 % to 67 %, 50 % to 90 %, 50 % to 80 %, 50 % to 70 %, 50 % to 67 %, or 60 % to 67 %, with respect to 100% of the colorectal cancer tumor volume of a negative control that was not administered with the strain and the anticancer agent.

In an embodiment, co-administration of the *Lactobacillus plantarum* GB104 strain, the cancer immunotherapeutic agent, and the chemotherapeutic agent may exhibit an activity that reduces the colorectal cancer tumor volume by 90 % or less, 80 % or less, 70 % or less, 67 % or less, 10 % to 90 %, 10 % to 80 %, 10 % to 70 %, 10 % to 67 %, 20 % to 90 %, 20 % to 80 %, 20 % to 70 %, 20 % to 67 %, 30 % to 90 %, 30 % to 80 %, 30 % to 70 %, 30 % to 67 %, 40 % to 90 %, 40 % to 80 %, 40 % to 70 %, 40 % to 67 %, 50 % to 90 %, 50 % to 80 %, 50 % to 70 %, 50 % to 67 %, or 60 % to 67 %, with respect to 100% of the colorectal cancer tumor volume of a negative control that was not administered with the strain and the anticancer agent.

In an embodiment, when the *Lactobacillus plantarum* GB104 strain, the cancer immunotherapeutic agent GI-101, and the chemotherapeutic agent 5-FU were co-administered as a triple combination in a tumor animal model transplanted with the mouse colorectal cancer cell line MC-38, a synergistic anticancer effect was shown compared with administration of the *Lactobacillus plantarum* GB104 strain alone or co-administration of the *Lactobacillus plantarum* GB104 strain and the cancer immunotherapeutic agent GI-101. Specifically, the effect of inhibiting the growth and progression of colorectal cancer tumors was confirmed. On average, compared with a control, the experimental group co-administered with a triple combination of GB104, the cancer immunotherapeutic agent, and the chemotherapeutic agent exhibited an effect of reducing the tumor volume by 10 % to 90 %.

Another aspect provides a health functional food for preventing or ameliorating cancer, which includes a first active substance including one or more selected from the group consisting of a *Lactobacillus plantarum* strain, a culture of the strain, a lysate of the strain, or an extract of the strain, the culture, and the lysate, and is administered in combination with a second active substance including a cancer immunotherapeutic agent as a first anticancer agent.

Another aspect provides a food composition for preventing or ameliorating cancer, which: includes, as active ingredients, a first active substance including one or more selected from the group consisting of a *Lactobacillus plantarum* strain, a culture of the strain, a lysate of the strain, or an extract of the strain, the culture, and the lysate, and a second active substance including a cancer immunotherapeutic agent as a first anticancer agent; or includes the first active substance as an active ingredient and is administered in combination with the second active substance.

The "strain," "first active substance," "second active substance," "first anticancer agent," "second anticancer agent," "anticancer activity," and "co-administration" are as described above.

In an embodiment, the food composition may be administered to mammals, including humans, via various routes. The administration method may be any commonly used method, for example, oral, dermal, intravenous, intramuscular or subcutaneous administration, and may preferably be oral administration.

In an embodiment, the composition may include a first oral formulation including the first active substance and a second oral formulation including the second active substance, and the first and second oral formulations may be administered orally.

In an embodiment, the health functional food may further include a sitologically acceptable carrier.

The term "sitologically acceptable" as used herein means exhibiting non-toxicity to cells or humans exposed to the compound.

The term "alleviation" as used herein may refer to any action that at least reduces a parameter related to the condition being treated, for example, the severity of a symptom. In this regard, the health functional food may be used before or after the onset of the disease, simultaneously with or separately from a drug for treatment, for the prevention or amelioration of cancer.

In the health functional food, the active ingredient may be added to food as it is or used together with other foods or food ingredients, and may be appropriately used according to conventional methods. The mixing amount of the active ingredient may be suitably determined depending on the purpose of use thereof (prevention or amelioration). In general, when manufacturing food or beverages, the health functional food may be added in an amount of about 15 wt% or less, more specifically about 10 wt% or less, with respect to the raw ingredients. However, in the case of long-term intake for the purpose of health and hygiene or health control, the amount may be below the above ranges.

The health functional food may be formulated as one selected from the group consisting of tablets, pills, powders, granules, fine particles, capsules, and liquid formulations by further including one or more of carriers, diluents, excipients and additives. Examples of foods to which a compound according to an aspect may be added include various foods, powders, granules, tablets, capsules, syrups, beverages, gum, tea, vitamin complexes, health functional foods, and the like.

Specific examples of the carriers, excipients, diluents and additives may be at least one selected from the group consisting of lactose, dextrose, sucrose, sorbitol, mannitol, erythritol, starch, acacia gum, calcium phosphate, alginate, gelatin, calcium phosphate, calcium silicate, microcrystalline cellulose, polyvinylpyrrolidone, cellulose, methylcellulose, water, sugar syrup, methylcellulose, methylhydroxy benzoate, propylhydroxy benzoate, talc, magnesium stearate, and mineral oil.

In addition to containing the active ingredient, the health functional food may contain other ingredients as essential ingredients without particular limitation. For example, like normal beverages, the health functional food may contain various flavoring agents or natural carbohydrates as additional ingredients. Examples of the natural carbohydrates may be: conventional sugars, for example, monosaccharides such as glucose or fructose, disaccharides such as maltose or sucrose, and polysaccharides such as dextrin or cyclodextrin; and sugar alcohols such as xylitol, sorbitol, or erythritol. As flavoring agents other than those described above, natural flavoring agents (thaumatin, stevia extract (e.g., rebaudioside A, glycyrrhizin, and the like)) and synthetic flavoring agents (saccharin, aspartame, and the like) may advantageously be used. The ratio of the natural carbohydrates may be appropriately determined through selection by one of ordinary skill in the art.

In addition to these additives, the health functional food according to an aspect may include various nutrients, vitamins, minerals (electrolytes), flavors such as synthetic flavors and natural flavors, colorants and enhancers (cheese, chocolate, and the like), pectic acid and salts thereof, alginic acid and salts thereof, organic acids, protective colloidal thickeners, pH adjusters, stabilizers, preservatives, glycerin, alcohol, carbonating agents used in carbonated beverages, and the like. These ingredients may be used independently or in combination, and the ratio of these additives may also be appropriately selected by one of ordinary skill in the art.

The health functional food may be provided in a mixture with conventionally known health functional foods for preventing or ameliorating cancer or other existing health functional foods. The health functional food for preventing or ameliorating cancer may be a conventionally known health functional food for preventing or ameliorating metabolic diseases, an existing health functional food, or a newly developed health functional food.

When the health functional food includes other health functional foods that have the effect of preventing or ameliorating cancer, it is important to mix these in minimum amounts that can achieve maximum efficacy without side effects, which may be readily determined by one of ordinary skill in the art.

The food composition for preventing or ameliorating cancer may include all types of functional foods, nutritional supplements, health foods, and food additives, and these types of food compositions may be prepared in various forms according to conventional methods known in the art.

The compositions of the present disclosure may be considered food supplements. Food supplements, also known as dietary supplements or nutritional supplements, may be considered other particular pharmaceutical products. Food supplements are prepared to supplement the diet and are intended to provide nutrients or beneficial ingredients that may not be consumed or may be consumed in insufficient quantities in a normal diet. Most food supplements are considered foods, but may also be considered drugs, natural health products, or nutraceutical products. In the sense of the present disclosure, food supplements include health functional foods. Food supplements are usually sold over the counter without prescription. When food supplements are prepared in the form of pills or capsules, these include the same excipients used in pharmaceuticals. However, food supplements may also be prepared in the form of foods fortified with some nutrients (e.g., infant formula). Thus, in specific embodiments, the composition of the present disclosure may be a food supplement.

Compositions according to the present disclosure may be administered as they are or mixed with suitable edible liquids or solids or administered as tablets, pills, capsules, lozenges, granules, powders, suspensions, sachets, or syrups, or may be freeze-dried in unit dose form. It may also be in the form of monodoses of the freeze-dried composition to be mixed in a separate liquid container provided together prior to administration.

The composition of the present disclosure may be included in various edible products and food products, such as dairy products, for infants. The term "edible product" as used herein includes, in its broadest sense, any product in any form capable of being consumed by an animal (e.g., a product that is able to be accepted by the sense organs). The term "food product" is understood to be an edible product that provides nutritional support to the body. In particular, food products of particular interest are food supplements and infant formulas. The food preferably includes a carrier material, such as oatmeal gruel, lactic acid fermented foods, resistant starch, dietary fibers, carbohydrates, proteins, and glycosylated proteins. In specific embodiments, bacterial cells of the present disclosure are homogenized with other ingredients such as cereals or powdered milk to constitute an infant formula.

Another aspect provides a kit for preventing or treating cancer, which: includes, as active ingredients, a first active substance including one or more selected from the group consisting of a *Lactobacillus plantarum* strain, a culture of the strain, a lysate of the strain, or an extract of the strain, the culture, and the lysate, and a second active substance including a cancer immunotherapeutic agent as a first anticancer agent; or includes the first active substance as an active ingredient and is administered in combination with the second active substance.

Another aspect provides a method of delivering a drug into a subject, including a step of administering a composition which: includes, as active ingredients, a first active substance including one or more selected from the group consisting of a *Lactobacillus plantarum* strain, a culture of the strain, a lysate of the strain, or an extract of the strain, the culture, and the lysate, and a second active substance including a cancer immunotherapeutic agent as a first anticancer agent; or includes the first active substance as an active ingredient and is administered in combination with the second active substance, to a subject in need thereof.

Another aspect provides a method of preventing or treating cancer, including a step of administering a composition which: includes, as active ingredients, a first active substance including one or more selected from the group consisting of a *Lactobacillus plantarum* strain, a culture of the strain, a lysate of the strain, or an extract of the strain, the culture, and the lysate, and a second active substance including a cancer immunotherapeutic agent as a first anticancer agent; or includes the first active substance as an active ingredient and is administered in combination with the second active substance, to a subject in need thereof.

The "strain," "first active substance," "second active substance," "first anticancer agent," "second anticancer agent," "anticancer activity," and "co-administration" are as described above.

The subject may be a subject suffering from cancer. In addition, the subject may be a mammal, and preferably, may be a human.

The administration route, dosage, and number of administrations of the *Lactobacillus Plantarum* GB104 strain and the anticancer agent may be administered to a subject in various ways and amounts depending on the condition of a patient, and the presence or absence of side effects, and the optimal administration method, dosage and number of administrations may be appropriately selected within appropriate ranges by one of ordinary skill in the art. In addition, the composition may be administered in combination with other drugs known to have therapeutic effects on cancer (e.g., anticancer agents described above) or physiologically active substances, in addition to the active ingredients described above, or may be formulated in the form of a combination preparation with other drugs.

### Advantageous Effects

Bacterial cells of a *Lactobacillus plantarum* strain, or a culture broth of the strain not only can inhibit the growth of cancer cells, but can also induce the apoptosis of cancer cells, and when administered in combination with an anticancer agent, exhibit a synergistic effect on inhibiting the growth of cancer cells and tumors, and thus can be effectively used to prevent, treat or ameliorate cancer in the form of a pharmaceutical composition or health functional food.

### Description of the Drawings

FIG. 1 is a graph showing the results of cell viability analysis after the human colorectal cancer cell line HCT116 was treated with each of the culture supernatants of GB104 and other strains.
FIG. 2 illustrates graphs showing the results of confirming the cell cycle after the human colorectal cancer cell line HCT116 was treated with each of the culture supernatants of GB104 and a comparative strain (WCFS1).
FIG. 3 illustrates graphs showing a cancer cell killing effect after the human colorectal cancer cell line HCT116 or the human breast cancer cell line MDA-MB-231 was treated with each of the culture supernatants of GB104 and a comparative strain (WCFS1).
FIG. 4 illustrates graphs showing a tumor formation inhibitory effect after the mouse colorectal cancer cell line MC-38, the lung cancer cell line LLC1, and the breast cancer cell line 4T1 were treated with a GB104 culture supernatant.
FIG. 5 is a graph showing the results of analyzing a synergistic effect by measuring the tumor volume after co-administration of GB104 and 5-FU in an allograft MC-38 mouse colorectal carcinoma model.
FIG. 6 illustrates graphs showing the results of comparative analysis through measurement of the tumor volume after administration of GB104 or 5-FU alone and co-administration thereof in an allograft MC-38 mouse colorectal carcinoma model.
FIG. 7 is a graph showing the results of analyzing a synergistic effect by measuring the tumor volume after co-administration of GB104 and anti-PD-1 in an allograft MC-38 mouse colorectal carcinoma model.
FIG. 8 illustrates graphs showing the results of comparative analysis through measurement of the tumor volume after administration of GB104 or anti-PD-1 alone and co-administration thereof in an allograft MC-38 mouse colorectal carcinoma model.
FIG. 9 is a graph showing the results of analyzing a synergistic effect by measuring the tumor volume after co-treatment with GB104 and bevacizumab in a xenograft HT-29 mouse colorectal carcinoma model.
FIG. 10 illustrates graphs showing the results of comparative analysis through measurement of the tumor volume after administration of GB104 or bevacizumab alone and co-administration thereof in a xenograft HT-29 mouse colorectal carcinoma model.
FIG. 11 is a graph showing the results of analyzing a synergistic effect by measuring the tumor volume after co-treatment with GB104 and cetuximab in a xenograft HT-29 mouse colorectal carcinoma model.
FIG. 12 illustrates graphs showing the results of comparative analysis through measurement of the tumor volume after administration of GB104 or cetuximab alone and co-administration thereof in a xenograft HT-29 mouse colorectal carcinoma model.
FIG. 13 is a graph showing the results of analyzing a synergistic effect by measuring the tumor volume and size after co-administration of GB104 and regorafenib in an allograft MC-38 mouse colorectal carcinoma model.
FIG. 14 illustrates graphs showing the results of comparative analysis through measurement of the tumor volume after administration of GB104 or regorafenib alone and co-administration thereof in an allograft MC-38 mouse colorectal carcinoma model.
FIG. 15 illustrates graphs showing the results of analyzing a synergistic effect by measuring the tumor volume and weight after co-treatment with GB104 and GI-101 in an allograft MC-38 mouse colorectal carcinoma model.
FIG. 16 illustrates graphs showing the results of comparative analysis through measurement of the tumor volume after administration of GB104 or GI-101 alone and co-administration thereof in an allograft MC-38 mouse colorectal carcinoma model.
FIG. 17 illustrates graphs showing the results of analyzing a synergistic effect by measuring tumor volume and tumor weight after co-treatment with GB104 and GI-102 in an allograft MC-38 mouse colorectal carcinoma model.
FIG. 18 illustrates graphs showing the results of comparative analysis through measurement of the tumor volume after administration of GB104 or GI-102 alone and co-administration thereof in an allograft MC-38 mouse colorectal carcinoma model.
FIG. 19 is a graph showing the results of analyzing a synergistic effect by measuring tumor volume after co-treatment with a triple combination of GB104, GI-101, and 5-FU in an allograft MC-38 mouse colorectal carcinoma model.
FIG. 20 illustrates graphs showing the results of comparative analysis through measurement of the tumor volume after administration of GB104, GI-101, or 5-FU alone and co-administration thereof in an allograft MC-38 mouse colorectal carcinoma model.

### Mode for Invention

Hereinafter, preferred examples will be presented to aid in understanding of the present disclosure. However, the following examples are provided only to more easily understand the present disclosure, and the content of the present disclosure is not limited by the following examples. Various modification can be made in examples, and thus examples are not limited to the examples disclosed below and can be implemented in various other forms.

### Example 1. Isolation and identification of Lactobacillus plantarum GB104 strain

The isolation and identification of a *Lactobacillus plantarum* GB104 strain were performed by methods described in Korean Patent Application No. 10-2020-0186738 and Korean Patent Application No. 10-2022-0080567. The above-described documents are incorporated herein by reference in their entirety.

Briefly, *Lactobacillus plantarum* GB104 was isolated from vaginal samples of healthy women who visited a hospital for the purpose of health examination. First, vaginal internal samples were taken with a cotton swab, pre-inoculated on a Rogosa SL (MRS) plate medium, and incubated in an anaerobic chamber at 37 °C for 48 hours. When bacterial colonies grew, single colonies were subcultured onto fresh MRS plates for pure isolation. After pure isolation, strains were cultured using MRS medium. Next, among the cultured strains, a *Lactobacillus plantarum* GB104 strain, which showed low cytotoxicity and inhibitory effect on fat cell accumulation, was finally selected. To identify the finally selected *Lactobacillus plantarum* GB104 strain, the 16S rRNA gene sequences obtained through PCR using primers targeting the 16S rRNA gene were analyzed by the Sanger sequencing method, and the 16s rRNA sequences of *Lactobacillus Plantarum* GB104 were represented as SEQ ID NO: 1. The inventors of the present disclosure named the GB104 strain as *"Lactobacillus plantarum* GB104" (accession number: KCTC 14107BP) and deposited the strain in the Korean Collection for Type Cultures (KCTC) located in the Korea Research Institute of Bioscience and Biotechnology on January 14, 2020. In addition, the strain name *Lactobacillus plantarum* was changed to *Lactiplantibacillus plantarum.* In the following examples, the changed strain names of existing strains are described interchangeably.

### Experimental Example 1. Confirmation of cancer cell viability and cancer cell killing effect according to treatment with culture supernatant of GB104 strain Confirmation of cancer cell viability according to treatment with culture supernatant of GB104 strain

In this experimental example, a human colorectal cancer cell line was treated with each of the culture supernatants of various *L. Plantarum* strains, including the *L*. *Plantarum* GB104 strain, and cell viability was screened through MTT analysis.

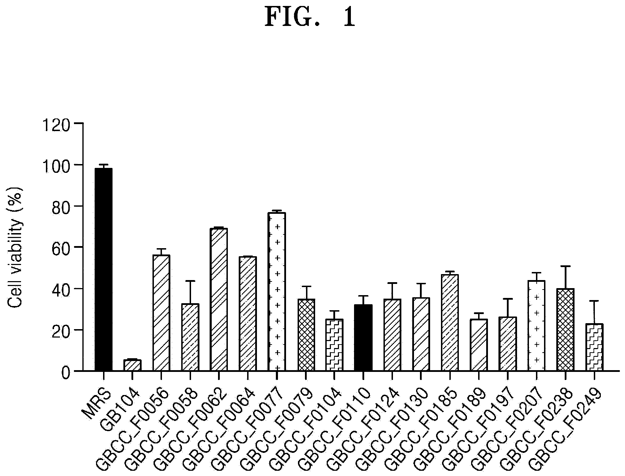

HCT116 cells, which are a human colorectal cancer cell line, were dispensed into each well of a 96-well plate at a concentration of 2X10³ cells, cultured for 24 hours, and then treated with culture supernatants of various *L. plantarum* strains at a concentration of 10 % and incubated at 37 °C in 5% CO₂ for 72 hours. To obtain the culture supernatant, the *L. plantarum* strain was cultured in MRS medium, and then the strain was precipitated by centrifugation to collect only the supernatant, followed by filtering through a 0.22 µm filter. The cells were treated with 0.5 mg/mL of an MTT solution by using cell proliferation kit I (MTT) (Roche), and then cultured further for 4 hours, and purple formazan crystals produced in living cells by metabolic activity were dissolved with a solubilization solution and absorbances at 570 nm were measured, and the results thereof are illustrated in FIG. 1.

FIG. 1 is a graph showing the results of cell viability analysis after the human colorectal cancer cell line HCT116 was treated with each of the culture supernatants of GB104 and other strains.

As illustrated in FIG. 1, the same cancer cell growth inhibitory effect was not shown in the various *L. plantarum* culture supernatants, and in particular, the GB104 culture supernatant exhibited an effect of reducing cancer cell viability by about 95 %, through which it was confirmed that the GB104 culture supernatant was most effective in inhibiting the growth of cancer cells.

### Confirmation of cell viability of human cancer cell lines according to treatment with GB104 strain culture supernatant

In this experimental example, various human cancer cell lines were each treated with a culture supernatant of the *L. plantarum* GB104 strain, and cell viability was screened through MTT analysis.

Human cancer cell lines were each dispensed into each well of a 96-well plate at a concentration of 0.8-1.5 x 10⁴ cells and cultured for 24 hours, and then colorectal cancer, lung cancer, gastric cancer, breast cancer, and liver cancer cell lines were treated with the culture supernatant of *L. plantarum* GB104 strain at a concentration of 10 %, and kidney cancer and bladder cancer cell lines were treated therewith at a concentration of 20 %. The cells were cultured for 48 hours under conditions of 37 °C and 5% CO2, the cell viability was measured in the same manner as in Experimental Example 1.1, and the results thereof are shown in Tables 1 and 2.

**[Table 1]**

| Carcinoma | | Cell line name | Cell viability (based on MRS 100 %) | |
|---|---|---|---|---|
| Colorectal cancer | | HCT116 | | 6.185±0.346 |
| Lung cancer | | A546 | | 9.761±3.399 |
| Gastric cancer | | MKN-45 | | 58.161±6.069 |
| Breast cancer | | MDA-MB-231 | | 66.969±4.102 |
| Liver cancer | | HepG2 | | 75.931±5.011 |

**[Table 2]**

| Carcinoma | | Cell line name | Cell viability (based on MRS 100 %) | |
|---|---|---|---|---|
| Kidney cancer | | A498 | | 5.630±0.226 |
| | | ACHN | | 4.790±0.042 |
| | | SN12C | | 6.185±0.247 |
| | | Caki-1 | | 5.360±0.071 |
| Bladder cancer | | RT-4 | | 5.710±0.184 |

As a result, it was confirmed that, although there are differences in cell viability depending on the characteristics of cancer cells, cancer cell lines treated with the *L. plantarum* GB104 culture supernatant, compared with the untreated control (MRS).

### 1.3. Confirmation of cancer cell cycle according to treatment with GB104 strain culture supernatant

In this experimental example, a human colorectal cancer cell line was treated with the *L. plantarum* GB104 culture supernatant, and a change in cancer cell cycle was examined by a flow cytometer.

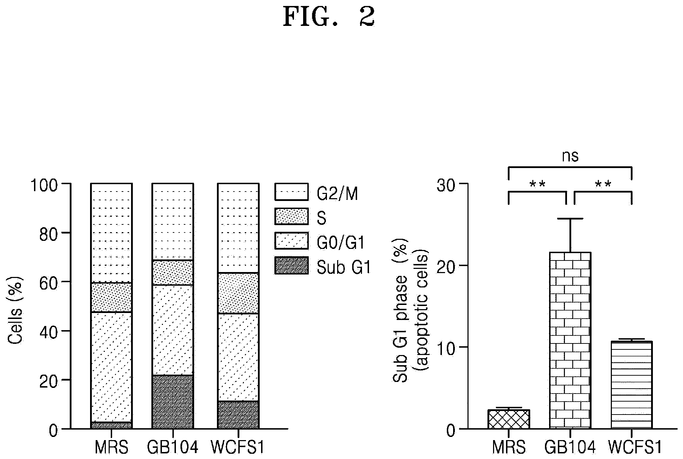

HCT116 cells, which are a human colorectal cancer cell line, were dispensed into each well of a 96-well plate at a concentration of 5X10⁴ cells, cultured for 24 hours, and then treated with a culture supernatant of the *L. plantarum* GB104 strain at a concentration of 10 % and incubated at 37 °C in 5 % CO₂ for 48 hours. To obtain the culture supernatant, the *L. plantarum* strain was cultured in MRS medium, and then the strain was precipitated by centrifugation to collect only the supernatant, followed by filtering through a 0.22 µm filter. After 48 hours, the cells were treated with trypsin-EDTA and removed, and then centrifuged to harvest cells, and the harvested cells were fixed with 70 % ethanol and stored at 4 °C for 1 hour or longer. After RNase A treatment and propidium iodine (PI) staining, the degree of apoptosis of cancer cells was confirmed through DNA content analysis using a flow cytometer (BD FACSymphony A3 Cell Analyzer), and the results thereof are illustrated in FIG. 2.

FIG. 2 illustrates graphs showing the results of confirming the cell cycle after the human colorectal cancer cell line HCT116 was treated with each of the culture supernatants of GB104 and a comparative strain (WCFS1).

As illustrated in FIG. 2, it was confirmed that the sub-G1 region of cells treated with the *L. plantarum* GB104 culture supernatant increased about 9.8 times compared with control cells treated with MRS. Through this, it was confirmed that GB104 induced the apoptosis of cancer cells.

### 1.4. Early and late cancer cell killing effects when human cancer cell lines were treated with GB104 culture supernatant

In this experimental example, human cancer cell lines were treated with the *L*. *plantarum* GB104 culture supernatant, and the cancer cell killing effects were examined by a flow cytometer.

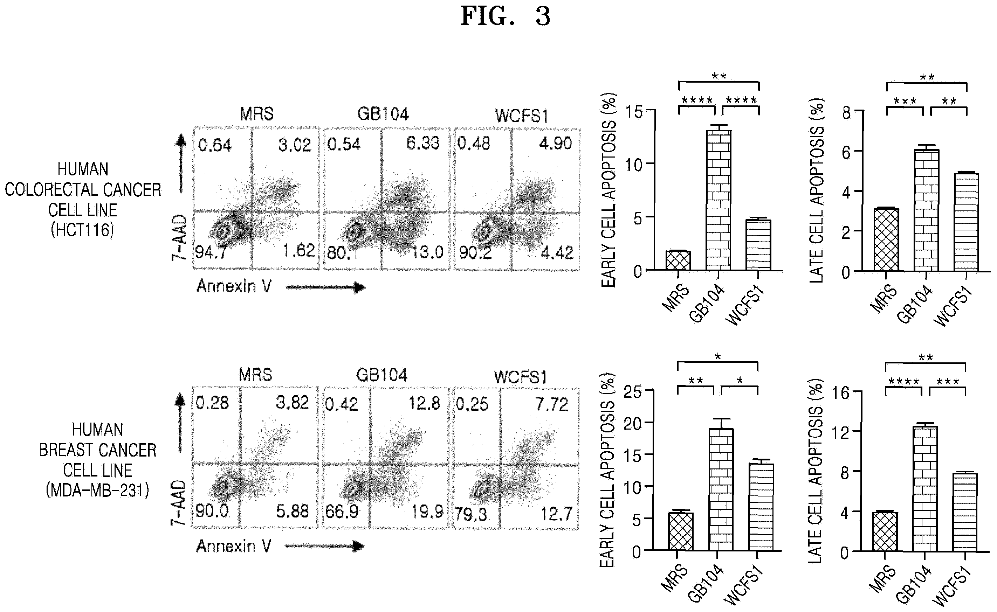

Human cancer cell lines were each dispensed into each well of a 6-well plate at a concentration of 0.5-1 x 10⁵ cells and cultured for 24 hours, and then treated with the culture supernatant of the *L. plantarum* GB104 strain at a concentration of 10 % and incubated at 37 °C in 5% CO₂ for 24 hours. To obtain the culture supernatant, the *L. plantarum* strain was cultured in MRS medium, and then the strain was precipitated by centrifugation to collect only the supernatant, followed by filtering through a 0.22 µm filter. After 24 hours, the cells were detached using trypsin-EDTA and harvested by centrifugation. The cells were mixed with 100 µL of a binding solution by using FITC Annexin V Apoptosis Detection Kit with 7-AAD (BioLegend), and then 5 µL of FITC Annexin V and 5 µL of 7-AAD were added and a reaction was allowed to occur for 15 minutes while being blocked from light at room temperature. Fluorescence measurement to confirm the apoptosis of cancer cells was performed by a flow cytometer (BD FACSymphony A3 Cell Analyzer), and the results thereof are illustrated in FIG. 3. Normally living cells are not labeled with Annexin V and 7-AAD, while cells in the early stages of apoptosis are labeled with Annexin V but not labeled with 7-AAD. Cells in the late stage of apoptosis can be identified by labeling the cells with both Annexin V and 7-AAD.

FIG. 3 illustrates graphs showing cancer cell killing effects after the human colorectal cancer cell line HCT116 or the human breast cancer cell line MDA-MB-231 was treated with each of the culture supernatants of GB104 and a comparative strain (WCFS1).

As illustrated in FIG. 3, it was confirmed that early apoptosis and late apoptosis were significantly increased in colorectal cancer and breast cancer cell lines treated with the *L. plantarum* GB104 culture supernatant, compared with the untreated control (MRS).

Through this, it was confirmed that GB104 is involved in inducing the apoptosis of cancer cells.

### Experimental Example 2. Tumor formation inhibitory effect according to treatment with GB104 culture supernatant

In this example, the inhibitory effect on tumor formation of *L. plantarum* GB104 strain treatment was examined in various mouse cancer cell lines.

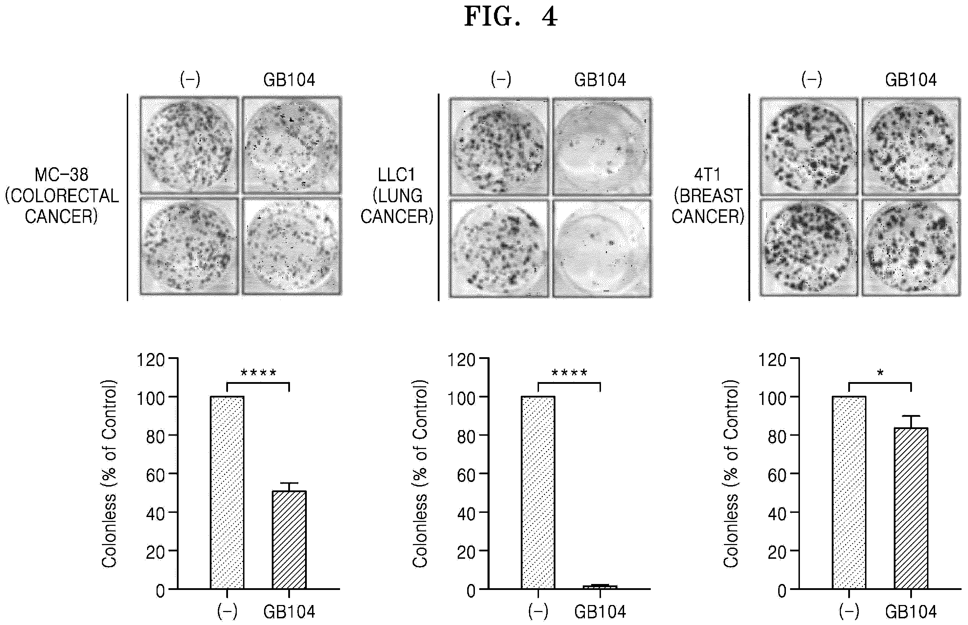

To obtain the culture supernatant, the *L. plantarum* strain was cultured in MRS medium, and then the strain was precipitated by centrifugation to collect only the supernatant, followed by filtering through a 0.22 µm filter. Specifically, each of the mouse colorectal cancer cell line MC-38, the mouse lung cancer cell line LLC1, and the mouse breast cancer cell line 4T1 was diluted in 2 mL of a medium at a concentration of 0.5-2 x 10³ cells in a 6-well plate, dispensed into each well, and stabilized for 24 hours. After 24 hours, the medium was removed and fresh medium replaced the medium, and each cancer cell line was treated with a culture supernatant of the *L. plantarum* GB104 strain at a concentration of 1 %. The medium was replaced with fresh medium containing the strain culture supernatant at intervals of 2 days to 3 days, and the cells were cultured for 7 days. The cultured plates were washed twice with DPBS, and the cells were fixed with 4% formalin. The fixed cells were washed twice with DPBS, stained with a 0.5% crystal violet solution for 5 minutes, and then washed with distilled water to confirm a colony forming ability, and the results thereof are illustrated in FIG. 4.

FIG. 4 illustrates graphs showing a tumor formation inhibitory effect after the mouse colorectal cancer cell line MC-38, the lung cancer cell line LLC1, and the breast cancer cell line 4T1 were each treated with the GB104 culture supernatant.

As illustrated in FIG. 4, it was confirmed that, although there are differences in the ability to inhibit tumor formation depending on the characteristics of cancer cells, tumor formation was inhibited in all the mouse cancer cell lines treated with the *L. plantarum* GB104 culture supernatant, compared with the untreated control (MRS).

### Experimental Example 3. Confirmation of tumor growth inhibitory effect according to co-administration of GB104 strain and chemotherapeutic agent 5-fluorouracil

In this experimental example, the tumor treatment effect of co-administration of the *L. plantarum* GB104 strain and 5-fluorouracil (5-FU) was examined in an allograft MC-38 colorectal carcinoma model. Six-week-old c57BL/6 mice were received and acclimatized for one week, and experiments were conducted at 7 weeks of age after hair removal from the right flank. A tumor model was established by subcutaneously injecting the c57BL/6-derived colorectal cancer cell line MC-38 into the right flank of each mouse at a concentration of 2 x 10⁵ cells/100 µL per mouse. Tumor volume was measured using a digital caliper and calculated by using tumor volume (mm³) = (width² x length)/2. On day 5 after tumor cell injection, only mice with tumor volumes within a certain range were selected and classified into groups to have equivalent mean tumor volumes, and then the *L. plantarum* GB104 strain was orally administered daily to the animal model at a concentration of 1 x 10⁹ CFU per mouse from day 6 until immediately before the experiments were completed. The chemotherapeutic agent 5-FU (10 mg/kg) was administered intraperitoneally once a week alone or in combination with GB104.

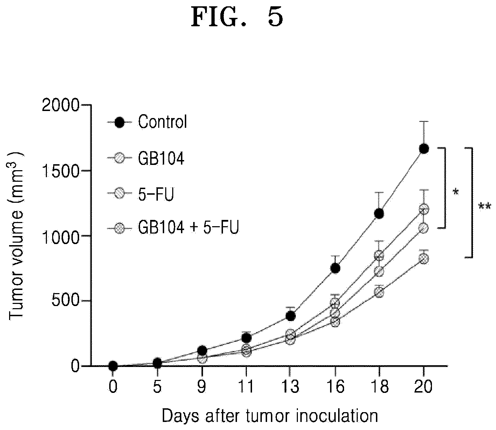

FIG. 5 is a graph showing the results of analyzing a synergistic effect by measuring the tumor volume after co-administration of GB104 and 5-FU in an allograft MC-38 mouse colorectal carcinoma model.

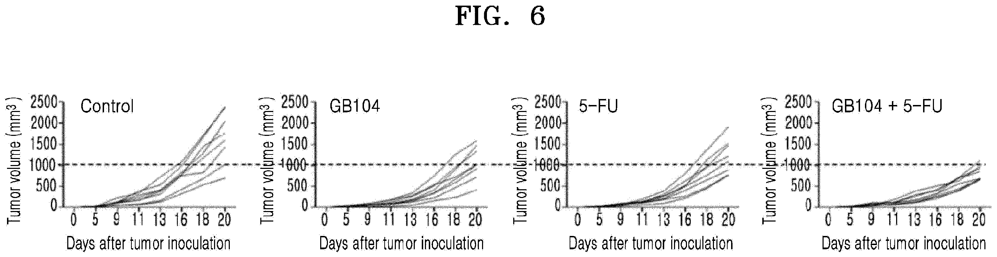

FIG. 6 illustrates graphs showing the results of comparative analysis through measurement of the tumor volume after administration of GB104 or 5-FU alone and co-administration thereof in an allograft MC-38 mouse colorectal carcinoma model.

As illustrated in FIGS. 5 and 6, it was confirmed that the tumor growth rate was significantly suppressed in the groups administered with *L. plantarum* GB104, compared with a negative control. It was also confirmed that the group co-administered with *L. plantarum* GB104 and 5-FU exhibited a significant reduction in tumor volume due to a synergistic phenomenon, compared with groups administered with *L. plantarum* GB104 or 5-FU alone.

### Experimental Example 4. Confirmation of tumor growth inhibitory effect according to co-administration of GB104 strain and cancer immunotherapeutic agent anti-PD-1

In this experimental example, the tumor treatment effect of co-administration of the *L. plantarum* GB104 strain and anti-PD-1 was examined in an allograft MC-38 colorectal carcinoma model. Six-week-old c57BL/6 mice were received and acclimatized for one week, and experiments were conducted at 7 weeks of age after hair removal around the right flank. A tumor model was established by subcutaneously injecting the c57BL/6-derived colorectal cancer cell line MC-38 into the right flank of each mouse at a concentration of 2 x 10⁶ cells/100 µL per mouse. Tumor volume was measured using a digital caliper and calculated by using tumor volume (mm³) = (width² x length)/2. On day 5 after tumor cell injection, only mice with tumor volumes within the range of 10 mm³ to 30 mm³ were selected and randomly grouped, and then the *L. plantarum* GB104 strain was orally administered daily to the animal model at a concentration of 1 x 10⁹ CFU per mouse from day 5 until immediately before the experiments were completed. The cancer immunotherapeutic agent anti-PD-1 (5 mg/kg) was administered intraperitoneally twice a week alone or in combination with GB104.

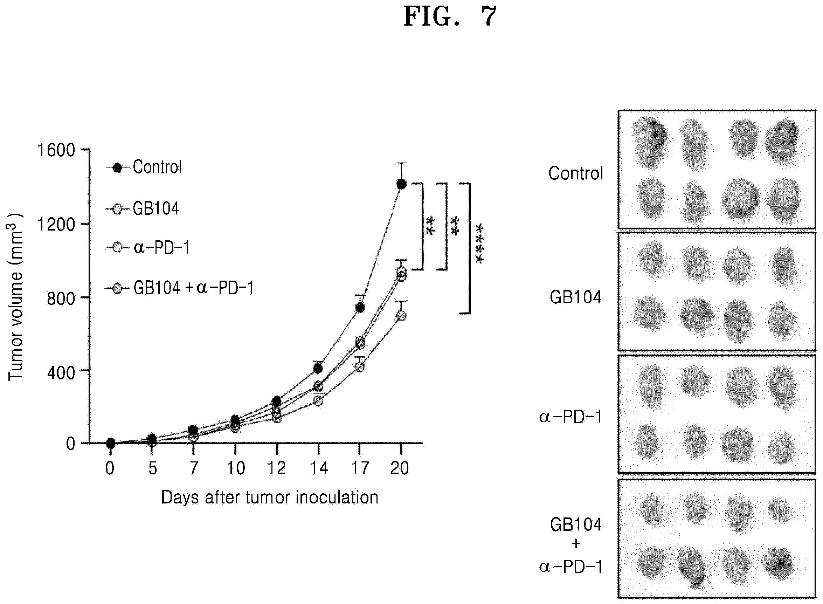

FIG. 7 is a graph showing the results of analyzing a synergistic effect by measuring the tumor volume after co-administration of GB104 and anti-PD-1 in an allograft MC-38 mouse colorectal carcinoma model.

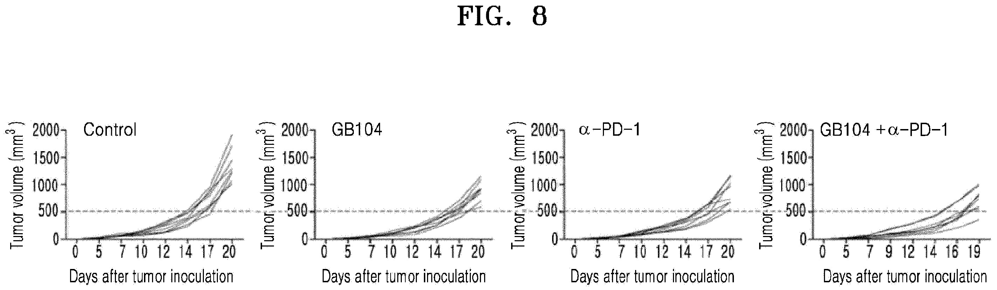

FIG. 8 illustrates graphs showing the results of comparative analysis through measurement of the tumor volume after administration of GB104 or anti-PD-1 alone and co-administration thereof in an allograft MC-38 mouse colorectal carcinoma model.

As illustrated in FIGS. 7 and 8, it was confirmed that the group co-administered with *L. plantarum* GB104 and anti-PD-1 exhibited a more significant reduction in tumor volume due to a synergistic phenomenon than groups administered with *L*. *plantarum* GB104 or anti-PD-1 alone, compared with a negative control.

### Experimental Example 5. Confirmation of tumor growth inhibitory effect according to co-administration of GB104 strain and targeted anticancer agent Bevacizumab

In this experimental example, the tumor treatment effect of co-administration of the *L. plantarum* GB104 strain and bevacizumab (anti-VEGF) was examined in a xenograft model using immunodeficient mice. Six-week-old NOG mice were received and acclimatized for one week, and experiments were conducted at 7 weeks of age after hair removal around the right flank. A tumor model was established by subcutaneously injecting the human colorectal cancer cell line HT-29 into the right flank of each mouse at a concentration of 3 x 10⁶ cells/100 µL per mouse. Tumor volume was measured using a digital caliper and calculated by using tumor volume (mm³) = (width² x length)/2. On day 3 after tumor cell injection, only mice with tumor volumes within a certain range were selected and classified into groups to have equivalent mean tumor volumes, and then the *L. plantarum* GB104 strain was orally administered daily to the animal model at a concentration of 1 x 10⁹ CFU per mouse from day 4 until immediately before the experiments were completed. The targeted anticancer agent Bevacizumab (10 mg/kg) was administered intraperitoneally once a week alone or in combination with GB104.

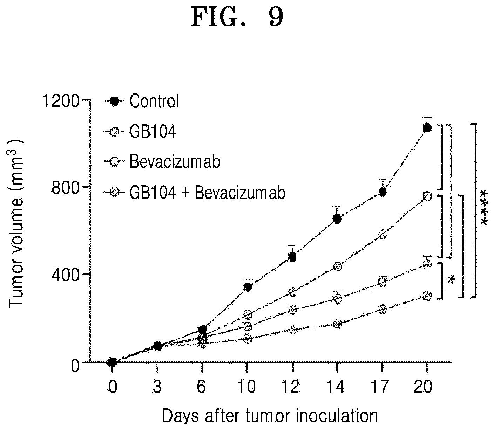

FIG. 9 is a graph showing the results of analyzing a synergistic effect by measuring the tumor volume after co-treatment with GB104 and bevacizumab in a xenograft HT-29 mouse colorectal carcinoma model.

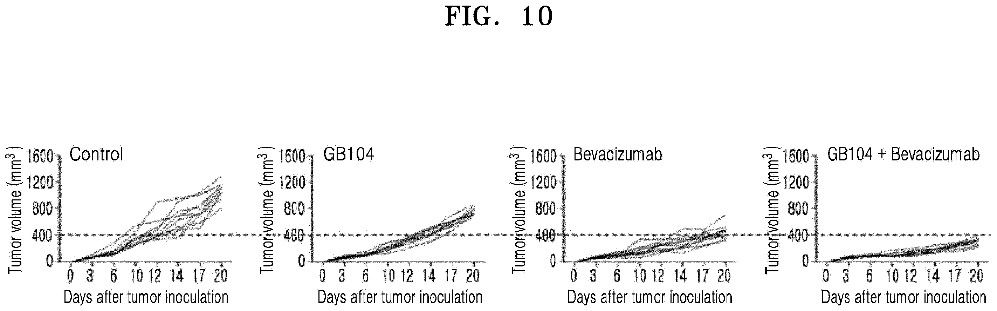

FIG. 10 illustrates graphs showing the results of comparative analysis through measurement of the tumor volume after administration of GB104 or bevacizumab alone and co-administration thereof in a xenograft HT-29 mouse colorectal carcinoma model.

As illustrated in FIGS. 9 and 10, it was confirmed that the tumor growth rate was suppressed in the groups administered with *L. plantarum* GB104, compared with a negative control. It was also confirmed that the group co-administered with *L. plantarum* GB104 and bevacizumab exhibited a more significant reduction in tumor volume due to a synergistic phenomenon than the groups administered with *L. plantarum* GB104 or bevacizumab alone.

### Experimental Example 6. Confirmation of tumor growth inhibitory effect according to co-administration of GB104 strain and targeted anticancer agent Cetuximab

In this experimental example, the tumor treatment effect of co-administration of the *L. plantarum* GB104 strain and cetuximab (anti-EGFR) was examined in a xenograft model using immunodeficient mice. Six-week-old NSG mice were received and acclimatized for one week, and experiments were conducted at 7 weeks of age after hair removal around the right flank. A tumor model was established by subcutaneously injecting the human colorectal cancer cell line HT-29 into the right flank of each mouse at a concentration of 3 x 10⁶ cells/100 µL per mouse. Tumor volume was measured using a digital caliper and calculated by using tumor volume (mm³) = (width² x length)/2. On day 3 after tumor cell injection, only mice with tumor volumes within a certain range were selected and classified into groups to have equivalent mean tumor volumes, and then the *L. plantarum* GB104 strain was orally administered daily to the animal model at a concentration of 1 x 10⁹ CFU per mouse from day 4 until immediately before the experiments were completed. The targeted anticancer agent Cetuximab (40 mg/kg) was administered intraperitoneally twice a week alone or in combination with GB104.

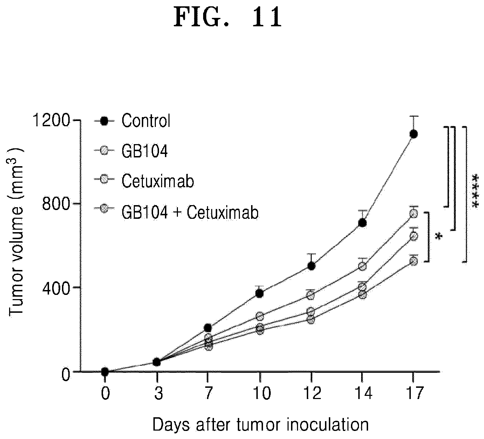

FIG. 11 is a graph showing the results of analyzing a synergistic effect by measuring the tumor volume after co-treatment with GB104 and cetuximab in a xenograft HT-29 mouse colorectal carcinoma model.

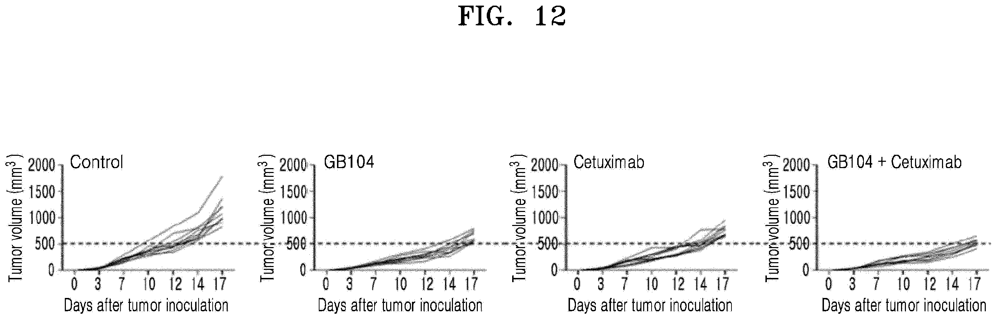

FIG. 12 illustrates graphs showing the results of comparative analysis through measurement of the tumor volume after administration of GB104 or cetuximab alone and co-administration thereof in a xenograft HT-29 mouse colorectal carcinoma model.

As illustrated in FIGS. 11 and 12, it was confirmed that the groups administered with *L. plantarum* GB104 exhibited a considerable tumor growth inhibitory effect, compared with a negative control. It was also confirmed that the group co-administered with *L. plantarum* GB104 and cetuximab exhibited the effect of reducing tumor volume due to a synergistic phenomenon, compared with a group administered with cetuximab alone.

### Experimental Example 7. Confirmation of tumor growth inhibitory effect according to co-administration of GB104 strain and targeted anticancer agent Regorafenib

In this experimental example, the tumor treatment effect of co-administration of the *L. plantarum* GB104 strain and regorafenib was examined in an allograft MC-38 colorectal carcinoma model. Five-week-old c57BL/6 mice were received and acclimatized for one week, and experiments were conducted at 6 weeks of age after hair removal around the right flank. A tumor model was established by subcutaneously injecting the c57BL/6-derived colorectal cancer cell line MC-38 into the right flank of each mouse at a concentration of 2 x 10⁵ cells/100 µL per mouse. Tumor volume was measured using a digital caliper and calculated by using tumor volume (mm³) = (width² x length)/2. On day 5 after tumor cell injection, only mice with tumor volumes within a certain range were selected and classified into groups to have equivalent mean tumor volumes, and then the *L. plantarum* GB104 strain was orally administered daily to the animal model at a concentration of 1 x 10⁹ CFU per mouse from day 5 until immediately before the experiments were completed. Regorafenib (3 mg/kg), which is a targeted anticancer drug, was administered orally alone or in combination with GB104 every other day from day 5 until immediately before the experiments were completed.

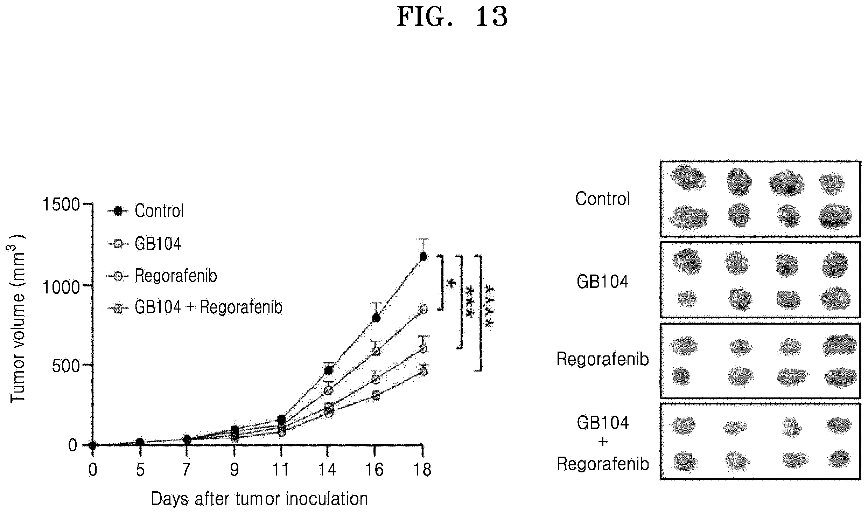

FIG. 13 is a graph showing the results of analyzing a synergistic effect by measuring the tumor volume and size after co-administration of GB104 and regorafenib in an allograft MC-38 mouse colorectal carcinoma model.

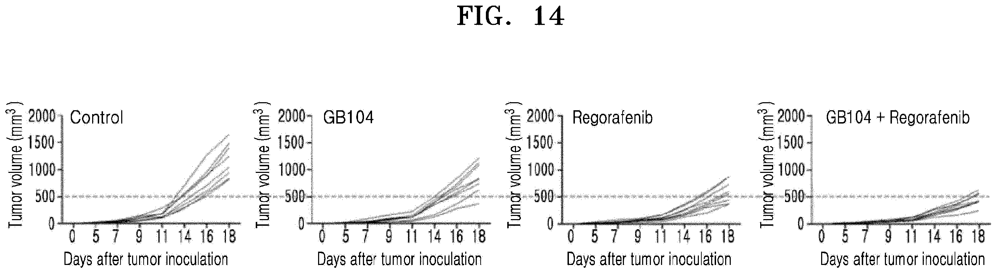

FIG. 14 illustrates graphs showing the results of comparative analysis through measurement of the tumor volume after administration of GB104 or regorafenib alone and co-administration thereof in an allograft MC-38 mouse colorectal carcinoma model.

As illustrated in FIGS. 13 and 14, it was confirmed that the tumor growth rate was significantly suppressed in the groups administered with *L. plantarum* GB104, compared with a negative control. It was also confirmed that the group co-administered with *L. plantarum* GB104 and regorafenib exhibited a significantly greater inhibitory effect on tumor volume, due to a synergistic phenomenon, than the groups administered with *L. plantarum* GB104 or regorafenib alone.

### Experimental Example 8. Confirmation of tumor growth inhibitory effect according to co-administration of GB104 strain and cancer immunotherapeutic agent GI-101

In this experimental example, the tumor treatment effect of co-administration of the *L. plantarum* GB104 strain and GI-101 was examined in an allograft MC-38 colorectal carcinoma model. Five-week-old c57BL/6 mice were received and acclimatized for one week, and experiments were conducted at 6 weeks of age after hair removal around the right flank. A tumor model was established by subcutaneously injecting the c57BL/6-derived colorectal cancer cell line MC-38 into the right flank of each mouse at a concentration of 2 x 10⁵ cells/100 µL per mouse. Tumor volume was measured using a digital caliper and calculated by using tumor volume (mm³) = (width² x length)/2. On day 5 after tumor cell injection, only mice with tumor volumes within the range of 10 mm³ to 30 mm³ were selected and randomly grouped, and then the *L. plantarum* GB104 strain was orally administered daily to the animal model at a concentration of 1 x 10⁹ CFU per mouse from day 5 until immediately before the experiments were completed. 3 mg/kg of GI-101, which is a cancer immunotherapeutic agent, was injected subcutaneously alone or in combination with GB104 once a week from day 5.

In this regard, the cancer immunotherapeutic agent GI-101 was prepared with reference to the content disclosed in Korean Patent Publication No. 10-2020-0032009. Specifically, to produce a fusion protein including a human CD80 fragment, an Fc domain, and an IL-2 variant, a polynucleotide including a nucleotide sequence (SEQ ID NO: 17) encoding a fusion protein which includes a signal peptide (SEQ ID NO: 16), a CD80 fragment (SEQ ID NO: 3), an Ig hinge (SEQ ID NO: 10), an Fc domain (SEQ ID NO: 9), a linker (SEQ ID NO: 11), and an IL-2 variant (2M) (R38A, F42A) (SEQ ID NO: 5) having two amino acid substitutions, in the stated order from the N-terminus was loaded into a pCGS3 vector (Sigma-Aldrich^{®}) by using a BioXPTM 3250 SYSTEM. The vector was introduced into CHO cells (Expi-CHO^{™}, Thermo Fisher Scientific) to express the fusion protein of SEQ ID NO: 12. After the vector was introduced, the cells were cultured for 7 days under conditions of 37 °C, 125 RPM, and a concentration of 8 % CO₂, and then the culture medium was collected to purify the fusion protein.

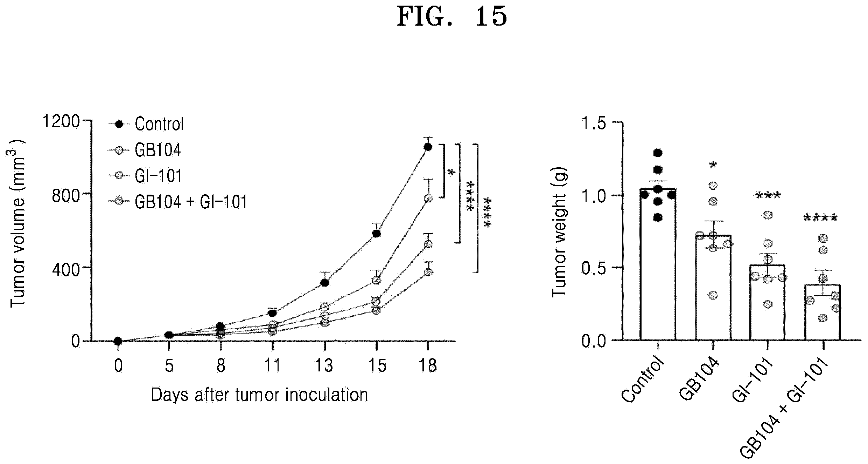

FIG. 15 illustrates graphs showing the results of analyzing a synergistic effect by measuring tumor volume and tumor weight after co-treatment with GB104 and GI-101 in an allograft MC-38 mouse colorectal carcinoma model.

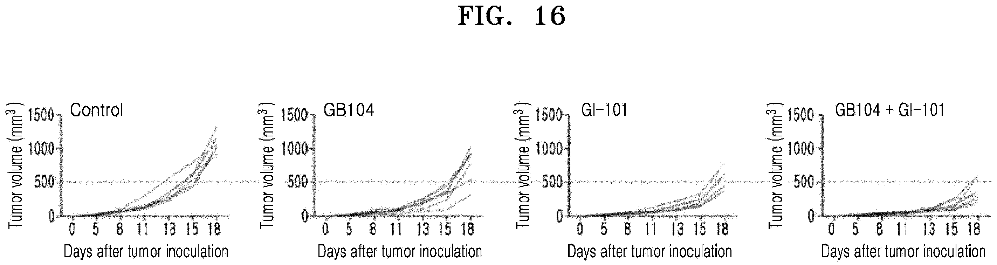

FIG. 16 illustrates graphs showing the results of comparative analysis through measurement of the tumor volume after administration of GB104 or GI-101 alone and co-administration thereof in an allograft MC-38 mouse colorectal carcinoma model.

As illustrated in FIGS. 15 and 16, it was confirmed that, compared with a negative control, more enhanced antitumor efficacy through a significant reduction in tumor volume was exhibited in the group co-administered with *L. plantarum* GB104 and GI-101 than in the groups administered with *L. plantarum* GB104 or GI-101 alone.

### Experimental Example 9. Confirmation of tumor growth inhibitory effect according to co-administration of GB104 strain and cancer immunotherapeutic agent GI-102

In this experimental example, the tumor treatment effect of co-administration of the *L. plantarum* GB104 strain and GI-102 was examined in an allograft MC-38 colorectal carcinoma model. Five-week-old c57BL/6 mice were received and acclimatized for one week, and experiments were conducted at 6 weeks of age after hair removal around the right flank. A tumor model was established by subcutaneously injecting the c57BL/6-derived colorectal cancer cell line MC-38 into the right flank of each mouse at a concentration of 2 x 10⁵ cells/100 µL per mouse. Tumor volume was measured using a digital caliper and calculated by using tumor volume (mm³) = (width² x length)/2. On day 5 after tumor cell injection, only mice with tumor volumes within the range of 10 mm³ to 30 mm³ were selected and randomly grouped, and then the *L. plantarum* GB104 strain was orally administered daily to the animal model at a concentration of 1 x 10⁹ CFU per mouse from day 5 until immediately before the experiments were completed. 3 mg/kg of GI-102, which is a cancer immunotherapeutic agent, was injected subcutaneously alone or in combination with GB104 on day 5 and on day 15.

In this regard, the cancer immunotherapeutic agent GI-102 was prepared with reference to the content disclosed in Korean Patent Publication No. 10-2020-0032009. Specifically, to produce a fusion protein including a human CD80 fragment, an Fc domain, and an IL-2 variant having three amino acid substitutions, a polynucleotide including a nucleotide sequence (SEQ ID NO: 18) encoding a fusion protein which includes a signal peptide (SEQ ID NO: 16), a CD80 fragment (SEQ ID NO: 3), an Ig hinge (SEQ ID NO: 10), an Fc domain (SEQ ID NO: 9), a linker (SEQ ID NO: 11), and an IL-2 variant having three amino acid substitutions (3M) (R38A, F42A, E61R) (SEQ ID NO: 7) in the stated order from the N-terminus, was loaded into a pCGS3 vector (Sigma-Aldrich^{®}) by using the BioXPTM 3250 SYSTEM. The vector was introduced into CHO cells (Expi-CHO^{™} Thermo Fisher Scientific) to express the fusion protein of SEQ ID NO: 14. After the vector was introduced, the cells were cultured for 7 days under conditions of 37 °C, 125 RPM, and a concentration of 8 % CO₂, and then the culture medium was collected to purify the fusion protein.

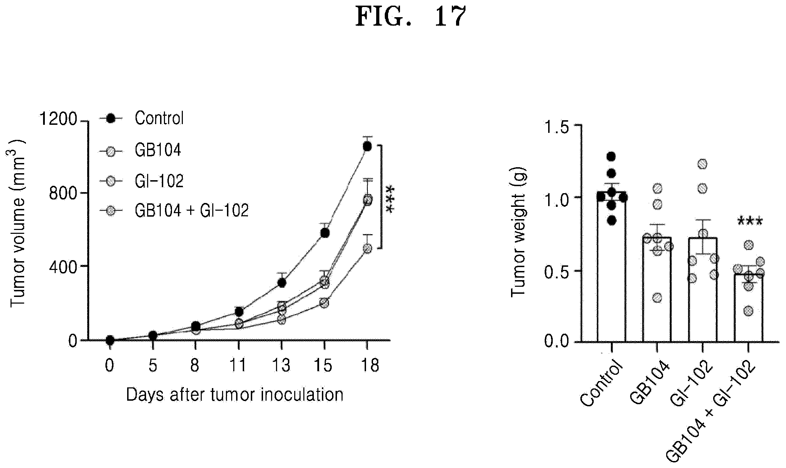

FIG. 17 illustrates graphs showing the results of analyzing a synergistic effect by measuring tumor volume and tumor weight after co-treatment with GB104 and GI-102 in an allograft MC-38 mouse colorectal carcinoma model.

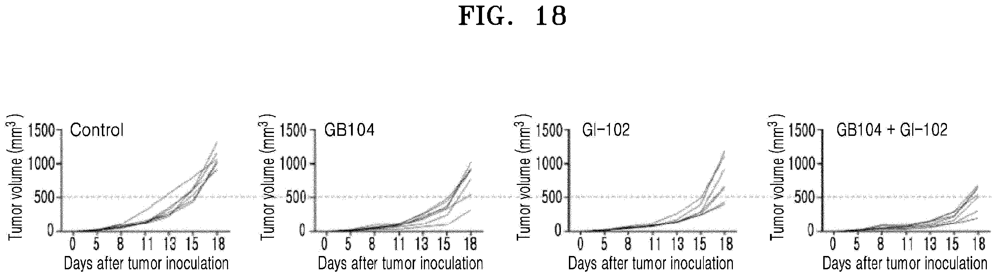

FIG. 18 illustrates graphs showing the results of comparative analysis through measurement of the tumor volume after administration of GB104 or GI-102 alone and co-administration thereof in an allograft MC-38 mouse colorectal carcinoma model.

As illustrated in FIGS. 17 and 18, it was confirmed that the tumor volume in the group co-administered with *L. plantarum* GB104 and GI-102 was significantly reduced compared with that in a negative control. It was also confirmed that the group co-administered with *L. plantarum* GB104 and GI-102 exhibited a significant reduction in tumor volume compared with the groups administered with L. *plantarum* GB104 or GI-102 alone. From the results, it was confirmed that, when *L. plantarum* GB104 and GI-102 were co-administered, the antitumor efficacy was enhanced.

### Experimental Example 10. Tumor growth inhibitory effect according to co-administration of GB104 strain, cancer immunotherapeutic agent GI-101, and chemotherapeutic agent 5-FU

In this experimental example, the tumor treatment effect of co-administration of the *L. plantarum* GB104 strain, the cancer immunotherapeutic agent GI-101, and a chemotherapeutic agent (5-fluorouracil, 5-FU) was examined in the allograft MC-38 colorectal carcinoma model. Six-week-old c57BL/6 mice were received and acclimatized for one week, and experiments were conducted at 7 weeks of age after hair removal around the right flank. A tumor model was established by subcutaneously injecting the c57BL/6-derived colorectal cancer cell line MC-38 into the right flank of each mouse at a concentration of 2 x 10⁵ cells/100 µL per mouse. Tumor volume was measured using a digital caliper and calculated by using tumor volume (mm³) = (width² x length)/2. On day 6 after tumor cell injection, only mice with tumor volumes within the range of 10 mm³ to 30 mm³ were selected and randomly grouped, and then the *L. plantarum* GB104 strain was orally administered daily to the animal model at a concentration of 1 x 10⁹ CFU per mouse from day 6 until immediately before the experiments were completed. 3 mg/kg of the cancer immunotherapeutic agent GI-101 was subcutaneously injected three times weekly from day 6. 10 mg/kg of the chemotherapeutic agent 5-FU was subcutaneously injected three times weekly from day 6.

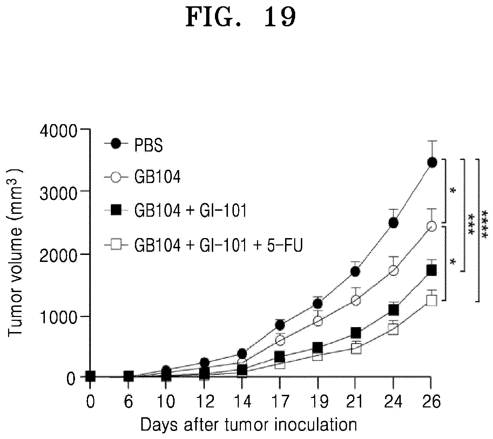

FIG. 19 is a graph showing the results of analyzing a synergistic effect by measuring the tumor volume after co-treatment with a triple combination of GB104, GI-101, and 5-FU in an allograft MC-38 mouse colorectal carcinoma model.

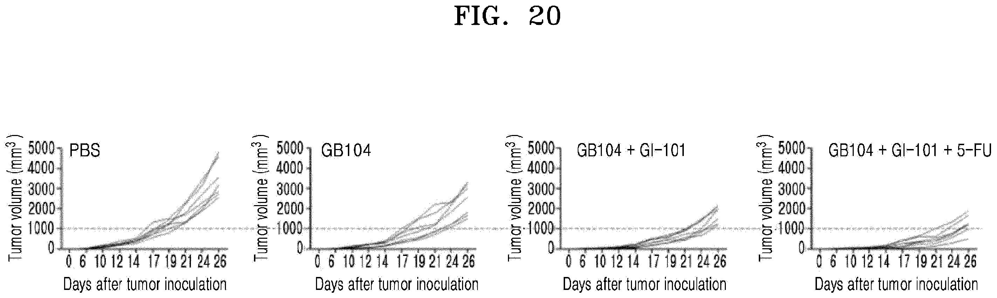

FIG. 20 illustrates graphs showing the results of comparative analysis through measurement of the tumor volume after administration of GB104, GI-101, or 5-FU alone and co-administration thereof in an allograft MC-38 mouse colorectal carcinoma model.

As illustrated in FIGS. 19 and 20, on day 26 after tumor implantation, the group administered with GB104 alone showed 29.72 % tumor growth inhibition, and the group co-administered with GB104 + GI-101 showed 50.01 % tumor growth inhibition, compared with a negative control (PBS). It was also confirmed that the group co-administered with a triple combination of GB104+GI-101+5-FU showed an excellent antitumor effect of 63.92 %.

From the above results, it was confirmed that, in colorectal cancer treatment, the groups administered with the GB104 strain alone and in combination with the cancer immunotherapeutic agent GI-101 and 5-FU, which is an existing chemotherapeutic agent, exhibited enhanced antitumor efficacy through significant inhibition of tumor growth.

### [Accession number]

Name of depository institution: Korea Research Institute of Bioscience and Biotechnology
Accession number: KCTC14107BP
Entrusted date: January 14, 2020

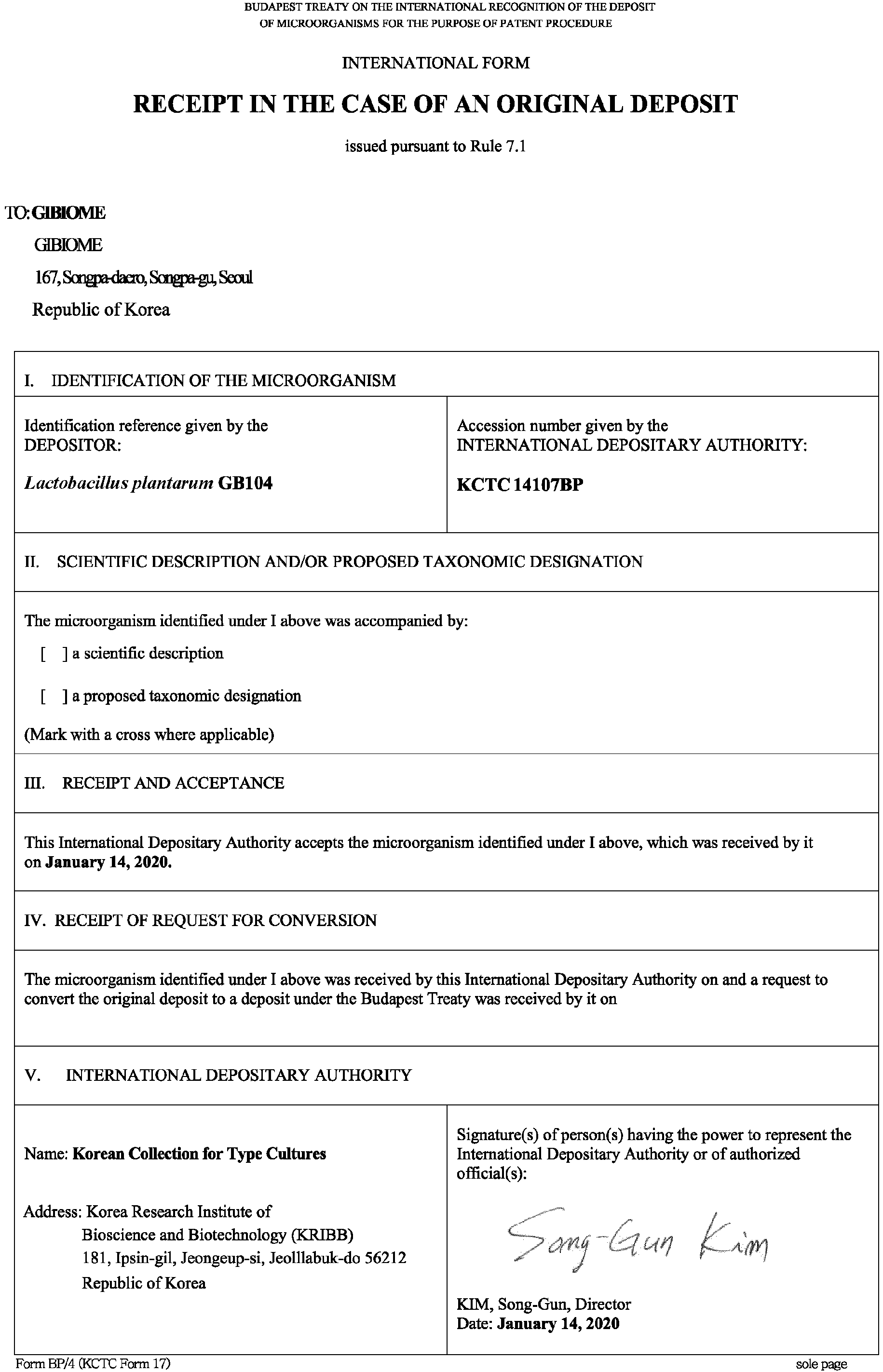
BUDAPEST TREATY ON THE INTERNATIONAL RECOGNITION OF THE DEPOSIT
OF MICROORGANISMS FOR THE PURPOSE OF PATENT PROCEDURE

INTERNATIONAL FORM

RECEIPT IN THE CASE OF AN ORIGINAL DEPOSIT

issued pursuant to Rule 7.1

TO: GIBIOME
GIBIOME
167, Songpa-daero, Songpa-gu, Seoul
Republic of Korea

I. IDENTIFICATION OF THE MICROORGANISM
Identification reference given by the
DEPOSITOR: | Accession number given by the
INTERNATIONAL DEPOSITARY AUTHORITY:
Lactobacillus plantarum GB104 | KCTC 14107BP

II. SCIENTIFIC DESCRIPTION AND/OR PROPOSED TAXONOMIC DESIGNATION

The microorganism identified under I above was accompanied by:
[ ] a scientific description
[ ] a proposed taxonomic designation
(Mark with a cross where applicable)

III. RECEIPT AND ACCEPTANCE

This International Depositary Authority accepts the microorganism identified under I above, which was received by it
on January 14, 2020.

IV. RECEIPT OF REQUEST FOR CONVERSION

The microorganism identified under I above was received by this International Depositary Authority on and a request to
convert the original deposit to a deposit under the Budapest Treaty was received by it on

V. INTERNATIONAL DEPOSITARY AUTHORITY

Name: Korean Collection for Type Cultures | Signature(s) of person(s) having the power to represent the
International Depositary Authority or of authorized
official(s):
Address: Korea Research Institute of
Bioscience and Biotechnology (KRIBB)
181, Ipsin-gil, Jeongeup-si, Jeolllabuk-do 56212
Republic of Korea |
KIM, Song-Gun, Director
Date: January 14, 2020

Form BP/4 (KCTC Form 17) sole page

## Claims

1. A pharmaceutical composition for preventing or treating cancer, comprises, as active ingredients, a first active substance comprising a *Lactobacillus plantarum* strain, a culture of the strain, a lysate of the strain, or a mixture thereof, and a second active substance comprising a cancer immunotherapeutic agent as a first anticancer agent; or comprises the first active substance as an active ingredient, and is administered in combination with the second active substance.

2. The pharmaceutical composition of claim 1, wherein the first active substance has tumor growth inhibitory activity or tumor metastasis inhibitory activity.

3. The pharmaceutical composition of claim 1, wherein the first active substance induces the apoptosis of cancer cells.

4. The pharmaceutical composition of claim 1, wherein the strain comprises the 16S rRNA of SEQ ID NO: 1.

5. The pharmaceutical composition of claim 1, wherein the strain is a strain deposited under accession number KCTC14107BP.

6. The pharmaceutical composition of claim 1, wherein the strain comprises a mutation of a naturally occurring *Lactobacillus plantarum* strain.

7. The pharmaceutical composition of claim 1, wherein the cancer immunotherapeutic agent is a fusion protein comprising a CD80 protein or a fragment thereof and an IL-2 protein or a variant thereof.

8. The pharmaceutical composition of claim 7, wherein the IL-2 variant has at least one amino acid substitution at positions 38, 42, 45, 61, and 72 in the amino acid sequence of SEQ ID NO: 4.

9. The pharmaceutical composition of claim 7, wherein the IL-2 variant has the amino acid sequences of SEQ ID NOs: 5 to 8.

10. The pharmaceutical composition of claim 7, wherein the fragment of CD80 has the amino acid sequence of SEQ ID NO: 3.

11. The pharmaceutical composition of claim 7, wherein the fusion protein consists of Structural Formula (I) or (II):
N'-X-[linker (1)]n-Fc domain-[linker (2)]m-Y-C' (I); and
N'-Y-[linker (1)]n-Fc domain-[linker (2)]m-X-C' (II),
wherein, in Structural Formulae (I) and (II),
N' is the N-terminus of the fusion protein,
C' is the C-terminus of the fusion protein,
X is a CD80 protein or a fragment thereof,
Y is an IL-2 protein or a variant thereof,
the linkers (1) and (2) are peptide linkers, and
n and m are each independently 0 or 1.

12. The pharmaceutical composition of claim 7, wherein the fusion protein has at least 90% sequence identity to the amino acid sequences of SEQ ID NOs: 12 to 15.

13. The pharmaceutical composition of claim 7, wherein two identical fusion proteins bind to form a homodimer.

14. The pharmaceutical composition of claim 1, wherein the first active substance and the second active substance are co-administered simultaneously, sequentially, or in reverse order.

15. The pharmaceutical composition of claim 1, wherein the first active substance and the second active substance are administered orally, intravenously, intraperitoneally, or subcutaneously.

16. The pharmaceutical composition of claim 1, wherein the first active substance is administered orally, and the second active substance is administered intravenously or subcutaneously.

17. The pharmaceutical composition of claim 1, wherein the first active substance and the second active substance are administered to a subject at intervals of 1 day to 10 days.

18. The pharmaceutical composition of claim 1, wherein the first active substance is administered to a subject at intervals of 1 day to 3 days, and the second active substance is administered to a subject at intervals of 5 days to 10 days.

19. The pharmaceutical composition of claim 1, wherein the second active substance further comprises a second anticancer agent, wherein the second anticancer agent is different from the first anticancer agent.

20. The pharmaceutical composition of claim 19, wherein the second anticancer agent is any one selected from the group consisting of a chemotherapeutic agent, a targeted anticancer agent, and a cancer immunotherapeutic agent.

21. The pharmaceutical composition of claim 20, wherein the chemotherapeutic agent is any one selected from the group consisting of an alkylating agent, a microtubule inhibitor, an antimetabolite, and a topoisomerase inhibitor.

22. The pharmaceutical composition of claim 20, wherein the chemotherapeutic agent is any one selected from the group consisting of mechlorethamine, cyclophosphamide, ifosfamide, melphalan, chlorambucil, thiotepa, altretamine, procarbazine, busulfan, streptozocin, carmustine, lomustine, dacarbazine, cisplatin, carboplatin, oxaliplatin, docetaxel, velban, oncovin, navelbine, 5-fluorouracil, capecitabine, cytarabine, gemcitabine, fludarabine, methotrexate, pemetrexed, mercaptopurine, hycamtin, camptosar, vepesid, paclitaxel, blenoxane, adriamycin, etoposide, and cerubidine.

23. The pharmaceutical composition of claim 20, wherein the targeted anticancer agent targets any one protein selected from the group consisting of epidermal growth factor receptor (EGFR), vascular endothelial growth factor receptor (VEGFR), CD20, CD38, RNAK-L, BTK, Bcr-abl, PDGFR/FGFR family, MEK/RAF, HER2/Neu, ubiquitin, JAK, MAP2K, ALK, PARP, tumor growth factor β receptor (TGFβR), proteasome, Bcl-2, C-Met, VR1, VR2, VR3, c-kit, AXL, RET, Braf, DNA methyltransferase (DNMT), CDK4/6, and STING.

24. The pharmaceutical composition of claim 20, wherein the targeted anticancer agent is any one selected from the group consisting of olaratumab, erlotinib, panitumumab, trastuzumab, trastuzumab emtansine, pertuzumab, cetuximab, rituximab, bevacizumab, axitinib, lenvatinib, ramucirumab, aflibercept, obinutuzumab, daratumumab, denosumab, ibrutinib, dasatinib, radotinib, nilotinib, imatinib, bosutinib, galunisertib, vactosertib, nintedanib, sunitinib, sorafenib, cabozantinib, regorafenib, masitinib, semaxanib, ceritinib, tivozanib, brigatinib, vandetanib, pazopanib, trametinib, temsirolimus, dabrafenib, dacomitinib, afatinib, lapatinib, neratinib, lenalidomide, osimertinib, ixazomib, olmutinib, everolimus, ruxolitinib, lestaurtinib, pacritinib, cobimetinib, selumetinib, binimetinib, bortezomib, alectinib, crizotinib, venetoclax, bemcentinib, gilteritinib, selpercatinib, pralsetinib, vemurafenib, olaparib, talazoparib, niraparib, rucaparib, azacitidine, decitabine, guadecitabine, gefitinib, abemaciclib, ribociclib, palbociclib, and DMXAA.

25. The pharmaceutical composition of claim 20, wherein the cancer immunotherapeutic agent is an antibody against any one selected from the group consisting of 2B4, 4-1BB (CD137), AaR, B7-H3, B7-H4, BAFFR, BTLA, CD2, CD7, CD27, CD28, CD30, CD40, CD80, CD83 ligand, CD86, CD160, CD200, CDS, CEACAM, CTLA-4, GITR, HVEM, ICAM-1, KIR, LAG-3, LAIR1, LFA-1 (CD 11 a/CD 18), LIGHT, NKG2C, NKp80, OX40, PD-1, PD-L1, PD-L2, SLAMF7, TGFRp, TIGIT, Tim3, and VISTA.

26. The pharmaceutical composition of claim 20, wherein the cancer immunotherapeutic agent is any one selected from the group consisting of atezolizumab, avelumab, durvalumab, nivolumab, pembrolizumab, abagovomab, adecatumumab, afutuzumab, alemtuzumab, anatumomab mafenatox, mepolizumab, blinatumomab, BMS-936559, catumaxomab, cemiplimab, epacadostat, epratuzumab, indoximod, inotuzumab, inotuzumab ozogamicin, intelumumab, ipilimumab, isatuximab, lambrolizumab, MED 14736, MPDL3280A, obinutuzumab, ocaratuzumab, ofatumumab, olatatumab, pidilizumab, rituximab, ticilimumab, samalizumab, and tremelimumab.

27. The pharmaceutical composition of claim 19, wherein the first anticancer agent and the second anticancer agent are co-administered simultaneously, sequentially, or in reverse order.

28. The pharmaceutical composition of claim 19, wherein the first anticancer agent and the second anticancer agent are administered orally, intravenously, intraperitoneally, or subcutaneously.

29. The pharmaceutical composition of claim 1, wherein the cancer is any one selected from the group consisting of gastric cancer, liver cancer, lung cancer, colorectal cancer, breast cancer, prostate cancer, ovarian cancer, pancreatic cancer, gallbladder cancer, biliary tract cancer, cervical cancer, thyroid cancer, laryngeal cancer, acute myeloid leukemia, brain tumor, neuroblastoma, retinoblastoma, salivary gland cancer, melanoma, bladder cancer, kidney cancer, hematologic malignancy, esophageal cancer, head and neck cancer, skin cancer, small intestine cancer, anal cancer, colon cancer, rectal cancer, and lymphoma.

30. The pharmaceutical composition of claim 20, wherein the colorectal cancer occurs in any one site selected from the group consisting of ascending colon, transverse colon, descending colon, sigmoid colon, and rectal mucosa.

31. A health functional food for preventing or ameliorating cancer, comprises, as active ingredients, a first active substance comprising a *Lactobacillus plantarum* strain, a culture of the strain, a lysate of the strain, or a mixture thereof, and a second active substance comprising a cancer immunotherapeutic agent as a first anticancer agent; or comprises the first active substance as an active ingredient, and is administered in combination with the second active substance.

32. The health functional food of claim 31, wherein the strain is a strain deposited under accession number KCTC14107BP.

33. The health functional food of claim 31, wherein the cancer immunotherapeutic agent is a fusion protein comprising an IL-2 protein or a variant thereof and a CD80 protein or a fragment thereof.

34. The health functional food of claim 31, wherein the second active substance further comprises a second anticancer agent, wherein the second anticancer agent is different from the first anticancer agent.

35. The health functional food of claim 34, wherein the second anticancer agent is any one selected from the group consisting of a chemotherapeutic agent, a targeted anticancer agent, and a cancer immunotherapeutic agent.

36. The health functional food of claim 31, wherein the first active substance and the second active substance are co-administered simultaneously, sequentially, or in reverse order.

37. The health functional food of claim 31, wherein the first active substance and the second active substance are administered orally, intravenously, intraperitoneally, or subcutaneously.

38. The health functional food of claim 31, wherein the first active substance is administered orally, and the second active substance is administered intravenously or subcutaneously.

39. The health functional food of claim 31, wherein the cancer is any one selected from the group consisting of gastric cancer, liver cancer, lung cancer, colorectal cancer, breast cancer, prostate cancer, ovarian cancer, pancreatic cancer, gallbladder cancer, biliary tract cancer, cervical cancer, thyroid cancer, laryngeal cancer, acute myeloid leukemia, brain tumor, neuroblastoma, retinoblastoma, salivary gland cancer, melanoma, bladder cancer, kidney cancer, hematologic malignancy, esophageal cancer, head and neck cancer, skin cancer, small intestine cancer, anal cancer, colon cancer, rectal cancer, and lymphoma.

40. The health functional food of claim 39, wherein the colorectal cancer occurs in any one site selected from the group consisting of ascending colon, transverse colon, descending colon, sigmoid colon, and rectal mucosa.

41. A method of preventing or treating cancer, comprising administering an effective amount of: a composition comprises, as active ingredients, a first active substance comprising a *Lactobacillus plantarum* strain, a culture of the strain, a lysate of the strain, or a mixture thereof, and a second active substance comprising a cancer immunotherapeutic agent as a first anticancer agent; or a composition comprising the first active substance as an active ingredient and administered in combination with the second active substance, to a subject in need thereof.

42. Use of: a composition comprising, as active ingredients, a first active substance comprising a *Lactobacillus plantarum* strain, a culture of the strain, a lysate of the strain, or a mixture thereof, and a second active substance comprising a cancer immunotherapeutic agent as a first anticancer agent; or a composition comprising the first active substance as an active ingredient and administered in combination with the second active substance, for preparing a pharmaceutical composition for preventing or treating cancer.

43. Use of: a composition comprising, as active ingredients, a first active substance comprising a *Lactobacillus plantarum* strain, a culture of the strain, a lysate of the strain, or a mixture thereof, and a second active substance comprising a cancer immunotherapeutic agent as a first anticancer agent; or a composition comprising the first active substance as an active ingredient and administered in combination with the second active substance, for preparing a health functional food for preventing or treating cancer.
